# EUROPEAN PATENT SPECIFICATION

(11) **EP 1 206 489 B1**
(45) Date of publication and mention of the grant of the patent: **06.05.2004**
(21) Application number: 00957607.5
(22) Date of filing: 17.08.2000
(51) Int. Cl.: C07K 14/475, A61K 38/18, A61P 25/28

(54) **ORALLY ACTIVE PEPTIDES THAT PREVENT CELL DAMAGE AND DEATH**
ORAL WIRKSAME PEPTIDE, DIE ZELLSCHÄDIGUNG UND ZELLTOD VERHINDERN
PEPTIDES ACTIFS PAR VOIE ORALE, EMPECHANT LA DETERIORATION ET LA MORT CELLULAIRES

(30) Priority: 18.08.1999 US 149956 P
(43) Date of publication of application: 22.05.2002
(73) Proprietor: RAMOT UNIVERSITY, AUTHORITY FOR APPLIED RESEARCH & INDUSTRIAL DEVELOPMENT LTD., Tel Aviv 61392 (IL); THE GOVERNMENT OF THE UNITED STATES OF AMERICA, represented by THE DEPARTMENT OF HEALTH & HUMAN SERVICES, Rockville, MD 20852 (US)
(72) Inventor: Brenneman, Douglas, E., Damascus, MD 20872 (US); Gozes, Illana, Ramat Hasharon (IL); Spong, Catherine, Y., Arlington, VA 22207 (US); Pinhasov, Albert, Egoz St., Tel Aviv (IL); Giladi, Eliezer, Ramat Poleg, Netania (IL)
(74) Representative: Heusch, Christian
(86) International application number: PCT/US2000/022861
(87) International publication number: WO 2001/012654

(56) References cited:
- WO-A-98/35042
- BRENNEMAN E.A.: "ADNF: structure-activity relationships of femtomolar-acting peptides" J.PHARM EXP.THERP, vol. 285, 1998, pages 619-627, XP002172069 cited in the application
- BASSAN M ET AL: "COMPLETE SEQUENCE OF A NOVEL PROTEIN CONTAINING A FEMTOMOLAR- ACTIVITY-DEPENDENT NEUROPROTECTIVE PEPTIDE" JOURNAL OF NEUROCHEMISTRY,US,NEW YORK, NY, vol. 72, no. 3, March 1999 (1999-03), pages 1283-1293, XP000882112 ISSN: 0022-3042 cited in the application

## Description

### FIELD OF THE INVENTION

This invention relates to Activity Dependent Neurotrophic Factor (ADNF) polypeptides comprising at least one D-amino acid within the active core sites of the ADNF polypeptides. The invention also relates to pharmaceutical compositions comprising ADNF polypeptides comprising at least one D-amino acid within the active core sites of the ADNF polypeptides. The invention further relates to the use of the ADNF polypeptides of the invention for the manufacture of a medicament for reducing neuronal cell death *in vitro* and *in vivo,* the use of the ADNF polypeptides of the invention for the manufacture of a medicament for treating oxidative stress in a patient, and the use of the ADNF polypeptides of the invention for the manufacture of a medicament for reducing a condition associated with fetal alcohol syndrome in a subject.

### BACKGROUND OF THE INVENTION

Neuronal cell death has been associated with various clinical conditions and diseases. These conditions and diseases include, for example, neurodegenerative diseases such as Alzheimer's disease, AIDS-related dementia, Huntington's disease, and Parkinson's disease. Neuronal cell death has been also associated with developmental retardation and learning impairments. These diseases and conditions are severely debilitating and have a lifelong impact on individuals diagnosed with such diseases and conditions.

It has previously been reported that Activity Dependent Neurotrophic Factor (ADNF) polypeptides can be used to prevent or reduce neuronal cell death. Activity Dependent Neurotrophic Factor I (ADNF I) polypeptide is secreted by astroglial cells in the presence of vasoactive intestinal peptide (VIP). The ADNF I polypeptide exhibits survival-promoting activity for neurons at surprisingly low, femtomolar concentrations (Brenneman & Gozes, *J*. *Clin. Invest.* 97:2299-2307 (1996)). Further studies identified peptide fragments of ADNF I that mimic the neurotrophic and neuroprotective properties of ADNF I. The shortest peptide (*i*.*e*., the active core site) that captured the survival-promoting activity of ADNF I was the peptide SALLRSIPA, designated as ADNF-9 or SAL (Brenneman *et al., J*. *Pharm. Exp. Therp.* 285:619-627 (1998)). Studies of related molecules to the ADNF I polypeptide resulted in the discovery of Activity Dependent Neuroprotective Protein (called ADNP or ADNF III interchangeably). This protein was cloned (Bassan *et al., J*. *Neurochem.* 72:1283-1293 (1999)) and was found to have an active peptide similar in biological activity to SAL. This peptide (*i*.*e*., the active core site) was NAPVSIPQ, designated as NAP.

ADNF polypeptides have been shown to prevent neuronal cell death both *in vitro* and *in vivo.* For example, ADNF polypeptides have been shown to prevent neuronal cell death associated with tetrodotoxin (electrical blockade), the β-amyloid peptide (the Alzheimer's disease neurotoxin), N-methyl-D-aspartate (excitotoxicity), and the human immune deficiency virus envelope protein. In addition, daily injections of ADNF polypeptides to newborn apolipoprotein E-deficient mice accelerated the acquisition of developmental reflexes and prevented short-term memory deficits. *See, e.g.,* Bassan *et al., J*. *Neurochem.* 72:1283-1293 (1999). Moreover, pretreatment with ADNF polypeptides has been previously shown to reduce numerous or various conditions associated with fetal alcohol syndrome in a subject. *See,* U.S.S.N. 09/265,511, filed March 12, 1999.

Although ADNF polypeptides have unlimited potential as neuroprotectants and/or therapeutic agents, it would be advantageous to provide additional ADNF polypeptides that have different properties from the known ADNF polypeptides. For example, availability of a number of ADNF polypeptides with different affinities for their receptors would allow targeting specific receptors in different cell types. Furthermore, additional ADNF polypeptides would aid in designing a drug treatment regime that can be individually tailored for each patient affected by neurodegenerative disorders.

### SUMMARY OF THE INVENTION

The present invention is based upon a surprising discovery that ADNF polypeptides comprising D-amino acids, which are not present in nature, are also effective for reducing neuronal cell death, for reducing oxidative stress, for reducing condition(s) associated with fetal alcohol syndrome in a subject, and for other conditions. The ADNF polypeptides include ADNF I and ADNF III polypeptides and subsequences thereof which contain their respective active core sites and provide neuroprotective and growth-promoting functions. The ADNF I polypeptides have an active core site comprising the following amino acid sequence: Ser-Ala-Leu-Leu-Arg-Ser-Ile-Pro-Ala ("SALLRSIPA" or "SAL"; SEQ ID NO:1). The ADNF III polypeptides also have an active core site comprising a few amino acid residues, namely, the following amino acid sequence: Asn-Ala-Pro-Val-Ser-Ile-Pro-Gln ("NAPVSIPQ" or "NAP"; SEQ ID NO:2). ADNF I polypeptides and ADNF III polypeptides comprising all L-amino acids have been previously shown to have remarkable potency and activity for reducing neuronal cell death *in vitro* and *in vivo,* as well as for reducing a condition associated with fetal alcohol syndrome in a subject.

As such, in one aspect, the present invention provides an Activity Dependent Neurotrophic Factor I (ADNF I) comprising an active core site having the following amino acid sequence: Ser-Ala-Leu-Leu-Arg-Ser-Ile-Pro-Ala (SEQ ID NO:1), wherein the active core site comprises at least one D-amino acid. In one embodiment, the N-terminal and/or the C-terminal amino acids of the active core site of the ADNF I polypeptide are D-amino acids. In another embodiment, the active core site of the ADNF I polypeptide comprises all D-amino acids. In another embodiment, an ADNF I polypeptide is Ser-Ala-Leu-Leu-Arg-Ser-Ile-Pro-Ala (SEQ ID NO:1), wherein the ADNF I polypeptide comprises at least one D-amino acid. In another embodiment, the ADNF I polypeptide is Ser-Ala-Leu-Leu-Arg-Ser-Ile-Pro-Ala (SEQ ID NO:1), wherein the ADNF I polypeptide comprises all D-amino acids.

In another aspect, the present invention provides an Activity Dependent Neurotrophic Factor III (ADNF III) comprising an active core site having the following amino acid sequence: Asn-Ala-Pro-Val-Ser-Ile-Pro-Gln (SEQ ID NO:2), wherein the active core site comprises at least one D-amino acid. In one embodiment, the N-terminal and/or the C-terminal amino acids of the active core site are D-amino acids. In another embodiment, the active core site of the ADNF III polypeptide comprises all D-amino acids. In another embodiment, the ADNF III polypeptide is Asn-Ala-Pro-Val-Ser-Ile-Pro-Gln (SEQ ID NO:2), wherein the ADNF III polypeptide comprises at least one D-amino acid. In another embodiment, the ADNF III polypeptide is Asn-Ala-Pro-Val-Ser-Ile-Pro-Gln (SEQ ID NO:2), wherein the ADNF III polypeptide comprises all D-amino acids.

In yet another aspect, the present invention provides a pharmaceutical composition comprising a pharmaceutically acceptable excipient and an ADNF polypeptide, wherein the ADNF polypeptide is a member selected from the group consisting of: (a) an ADNF I polypeptide comprising an active core site having the following amino acid: Ser-Ala-Leu-Leu-Arg-Ser-Ile-Pro-Ala (SEQ ID NO:1); (b) an ADNF III polypeptide comprising an active core site having the following amino acid: Asn-Ala-Pro-Val-Ser-Ile-Pro-Gln (SEQ ID NO:2); and (c) a mixture of the ADNF I polypeptide or part (a) and the ADNF III polypeptide of part (b); wherein at least one of the ADNF I polypeptide and the ADNF III polypeptide comprises an active core site comprising at least one D-amino acid.

In one embodiment, the pharmaceutical composition comprises an ADNF I polypeptide, wherein the ADNF I polypeptide comprises all D-amino acids. In another embodiment, the pharmaceutical composition comprises an ADNF III polypeptide, wherein the ADNF III polypeptide comprises all D-amino acids. In another embodiment, the pharmaceutical composition comprises an ADNF I polypeptide and an ADNF III polypeptide, wherein the ADNF I polypeptide and the ADNF III polypeptide both comprise all D-amino acids. In another embodiment, the pharmaceutical composition comprises an ADNF I polypeptide and an ADNF III polypeptide, wherein the ADNF I polypeptide comprises all D-amino acids and wherein the ADNF III polypeptide comprises all L-amino acids. In another embodiment, the pharmaceutical composition comprises an ADNF I polypeptide and an ADNF III polypeptide, wherein the ADNF I polypeptide comprises all L-amino acids and wherein the ADNF III polypeptide comprises all D-amino acids.

In yet another aspect, the present invention provides the use of the ADNF polypeptides of the invention for the manufacture of a medicament for preventing neuronal cell death, the method comprising contacting neuronal cells with at least one of the above described ADNF polypeptides. In one embodiment, the neuronal cell death is in a patient infected with immunodeficiency virus. In another embodiment, the neuronal cell death is associated with excito-toxicity induced by N-methyl-D-aspartate stimulation. In yet another embodiment, the neuronal cell death is induced by the beta-amyloid peptide in a patient afflicted with Alzheimer's disease. In yet another embodiment, the neuronal cell death is induced by cholinergic blockade in a patient afflicted with Alzheimer's disease, which results in learning impairment.

In yet another aspect, the present invention provides the use of the ADNF polypeptides of the invention for the manufacture of a medicament for reducing oxidative stress in a patient, the method comprising administrating to the patient at least one of the ADNF polypeptides described above in an amount sufficient to treat oxidative stress.

In yet another aspect, the present invention provides the use of the ADNF polypeptides of the invention for the manufacture of a medicament for reducing a condition associated with fetal alcohol syndrome in a subject who is exposed to alcohol *in utero,* the method comprising administering to the subject at least one ADNF polypeptides described above in an amount sufficient to reduce a condition associated with fetal alcohol syndrome.

### BRIEF DESCRIPTION OF THE DRAWINGS

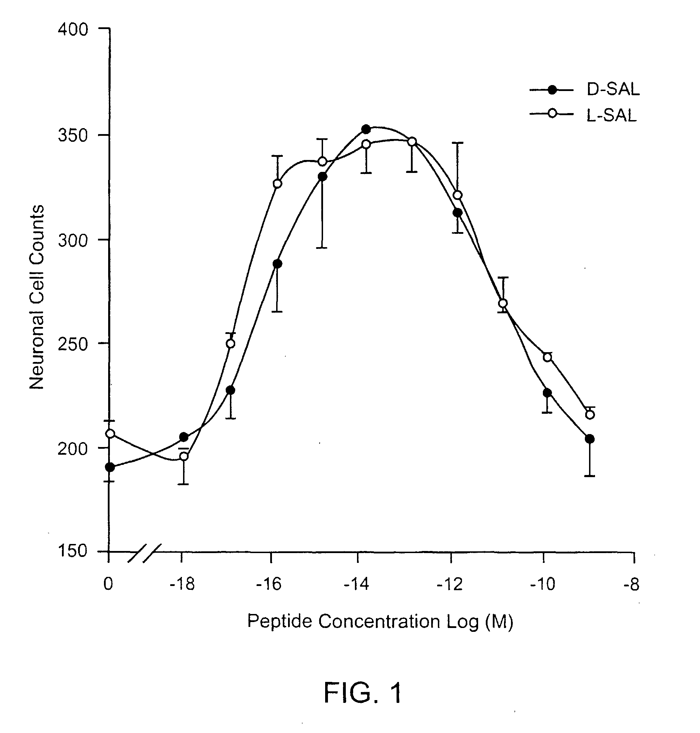

Figure 1 compares the survival-promoting activity of D- and L-forms of SALLRSIPA in dissociated cerebral cortical cultures treated with 1 µM tetrodotoxin, an agent that blocks electrical activity and produces apoptotic neuronal cell death. Treatment duration was for 5 days. Each point is the mean ± the standard error of 3-4 determinations. Neuronal cell counts were obtained without knowledge of the treatment group.

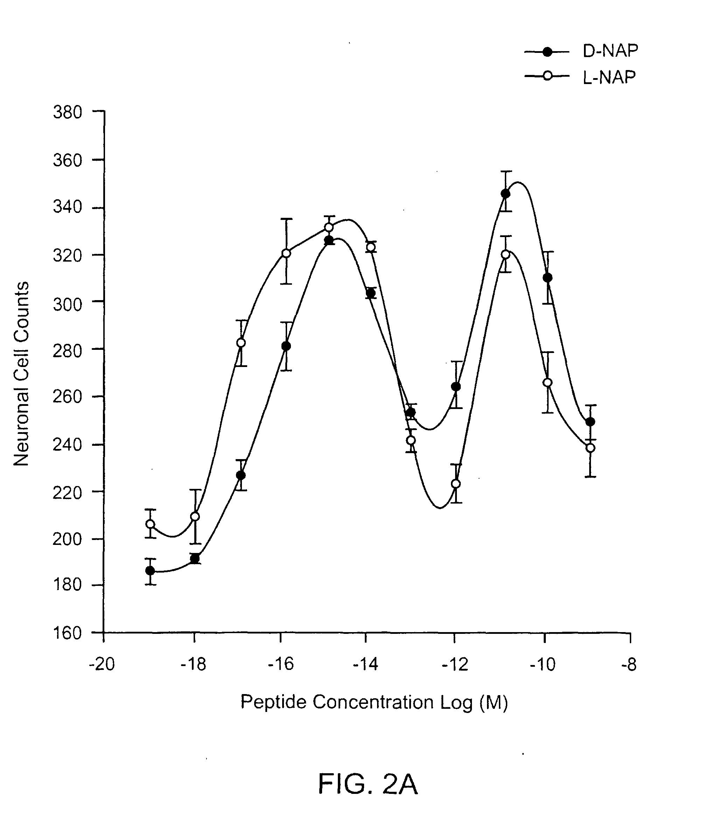

Figure 2A compares the survival-promoting activity of D- and L-forms of NAPVSIPQ in dissociated cerebral cortical cultures treated with 1 µM tetrodotoxin. Experimental conditions were as described for Figure 1.

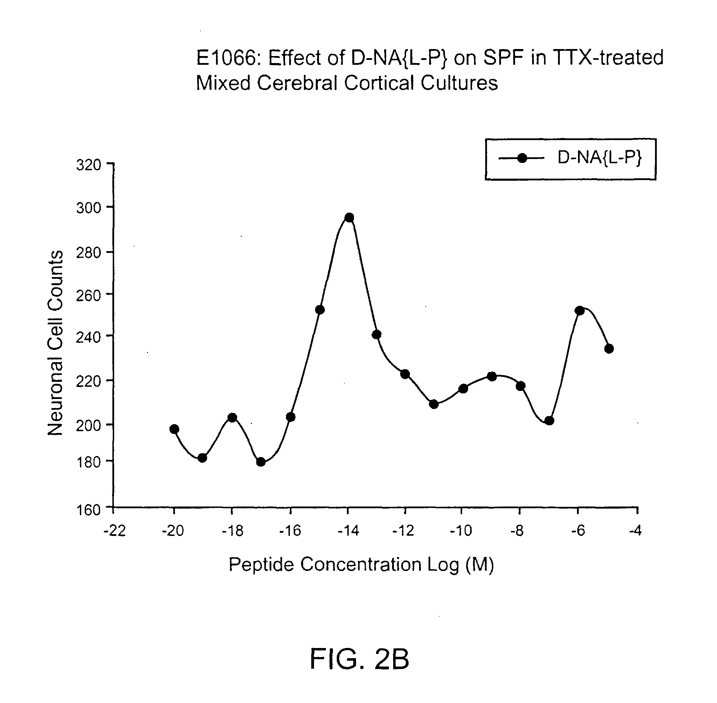

Figure 2B illustrates the effect of a mixture of D- and L-amino acid D-NA{L-P}VSIPQ on survival promoting activity in cerebral cortical culture co-treated with tetrodotoxin for 5 days. In peptide NAPVSIPQ, all of the amino acids were in the D-form, except the third proline residue was in the L-form.

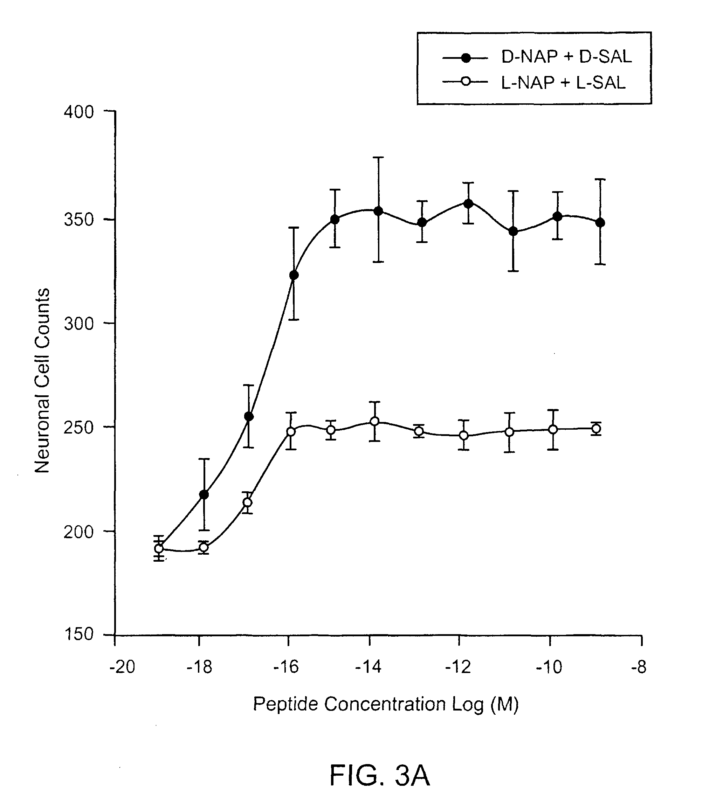

Figure 3A compares the survival-promoting activity of combinations of NAPVSIPQ and SALLRSIPA in D- and L-forms. Experimental conditions were as described for Figure 1.

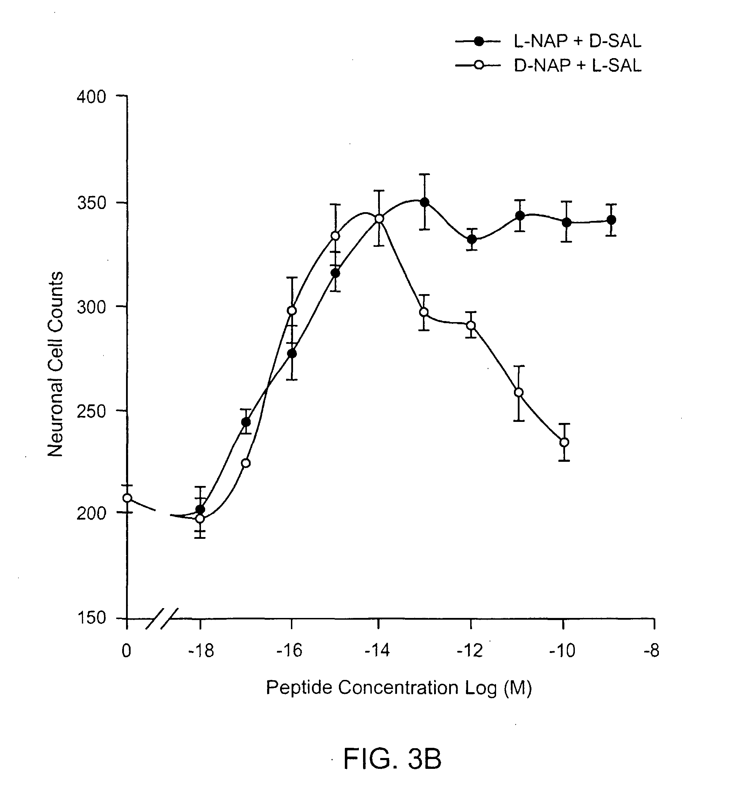

Figure 3B compares the survival-promoting activity of combinations of L-NAPVSIPQ + D-SALLRSIPA with D-NAPVSIPQ and L-SALLRSIPA. Experimental conditions were as described for Figure 1.

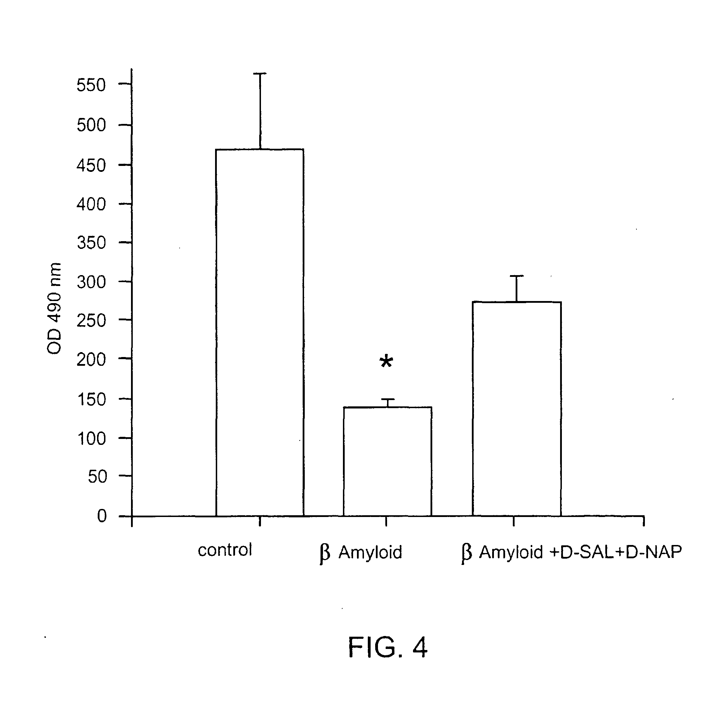

Figure 4 illustrates that combinations of D-SALLRSIPA + D-NAPVSIPQ can protect against beta amyloid toxicity in PC12 cells.

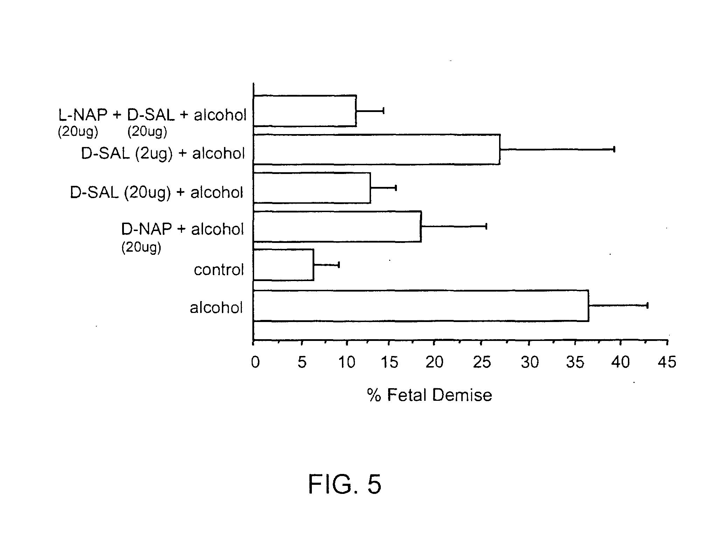

Figure 5 illustrates that pretreatment with D-NAP, D-SAL, or L-NAP + D-SAL prevents fetal demises. At E18, the number of living and demised embryos was counted and the percentage of demises was calculated. Treatment with alcohol was given on E8, pretreatment with peptides given 30 minutes prior. Comparisons are made to the alcohol group, overall ANOVA p< 0.001. Post hoc Fishers tests were performed, with the * groups significantly different than alcohol (all post-hoc p≤0.03). The sample sizes were control (36), alcohol (41), D-NAP (20 µg) + alcohol (14), D-SAL (20 µg) + alcohol (19), D-SAL (2 µg) + alcohol (8), L-NAP (20 µg) + D-SAL (20 µg) + alcohol (23).

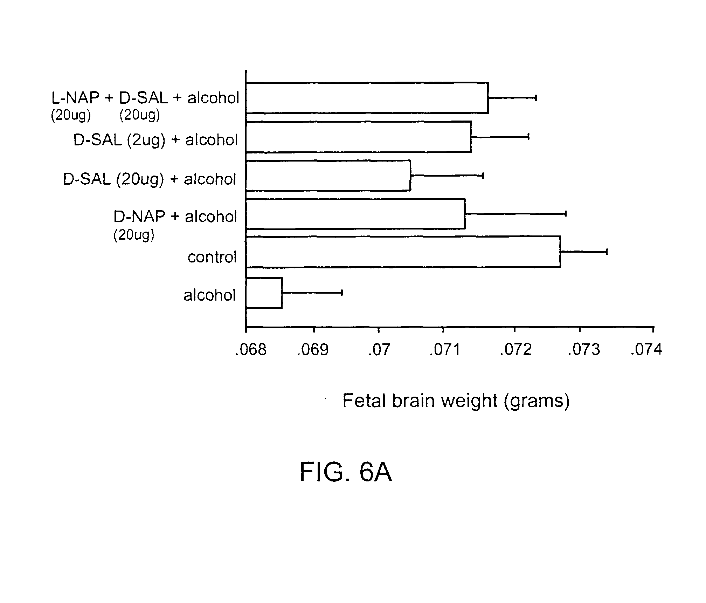

Figure 6A illustrates that pretreatment with L-NAP + D-SAL prevented fetal microcephaly. Fetal brain weights for each pregnant female were obtained at E18. Comparisons are made to the alcohol group, overall ANOVA P value p=0.01. Sample size was the number of litters. The mean from each litter was used for statistical analysis and represents on average 8-10 fetuses. The sample sizes were control (34), alcohol (32), D-NAP (20 µg) + alcohol (13), D-SAL (20 µg) + alcohol (19), D-SAL (2 µg) + alcohol (8), L-NAP (20 µg) + D-SAL (20 µg) + alcohol (23).

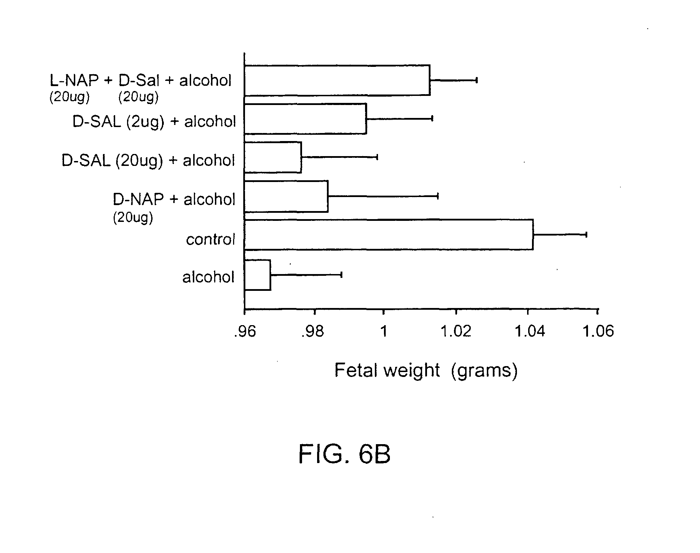

Figures 6B illustrates that pretreatment with L-NAP + D-SAL prevented or reduced fetal growth restriction. Fetal weights for each pregnant female were obtained at E18. Comparisons are made to the alcohol group, overall ANOVA P value p=.04. Sample size was the number of litters. The mean from each litter was used for statistical analysis and represents on average 8-10 fetuses. The sample sizes were control (34), alcohol (32), D-NAP (20 µg) + alcohol (13), D-SAL (20 µg) + alcohol (19), D-SAL (2 µg) + alcohol (8), L-NAP (20 µg) + D-SAL (20 µg) + alcohol (23).

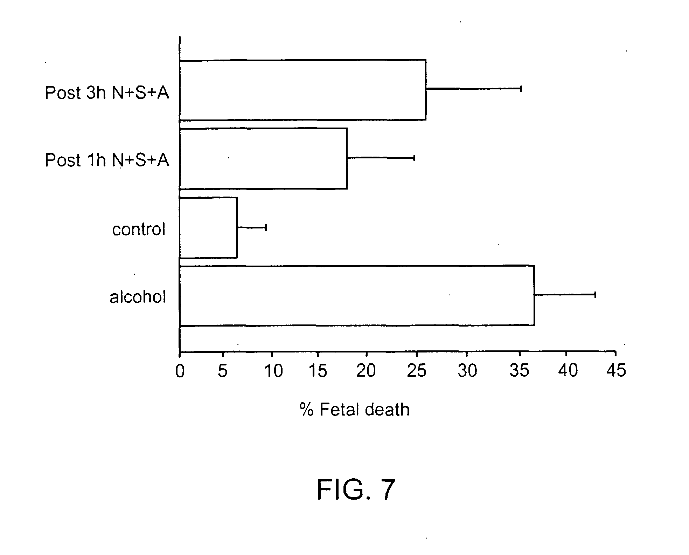

Figure 7 illustrates that one hour post-treatment with L-NAP and L-SAL prevented fetal death. L-NAP (20 µg) and L-SAL (20 µg) were given at one and three hours after alcohol administration on E8. Comparisons are made to the alcohol group, overall ANOVA p=0.001. Post hoc Fishers tests were performed, with the one-hour and control groups significantly different than alcohol (p<0.001 and p=0.04, respectively). The sample sizes were control (36), alcohol (41), post one hour treatment (18) and post three hour treatment (14).

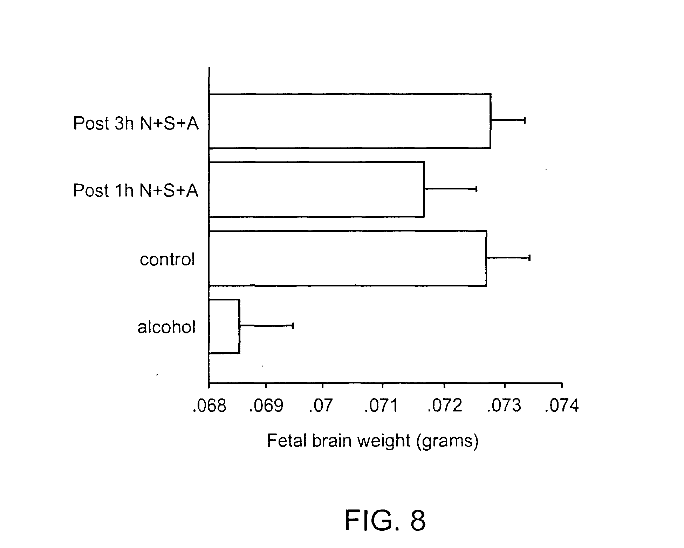

Figure 8 illustrates that one and three hour post-treatments with L-NAP and L-SAL prevented fetal microcephaly. L-NAP and L-SAL were given at one and three hours after alcohol administration on E8 (N+S+A). Comparisons are made to the alcohol group, overall ANOVA P=0.001. Post hoc Fishers tests were performed, with the one-hour, three hour, and control groups significantly different than alcohol (p<0.001, <0.03 and P<0.008 respectively). The sample sizes were control (34), alcohol (32), post one hour treatment (17) and post three hour treatment(11).

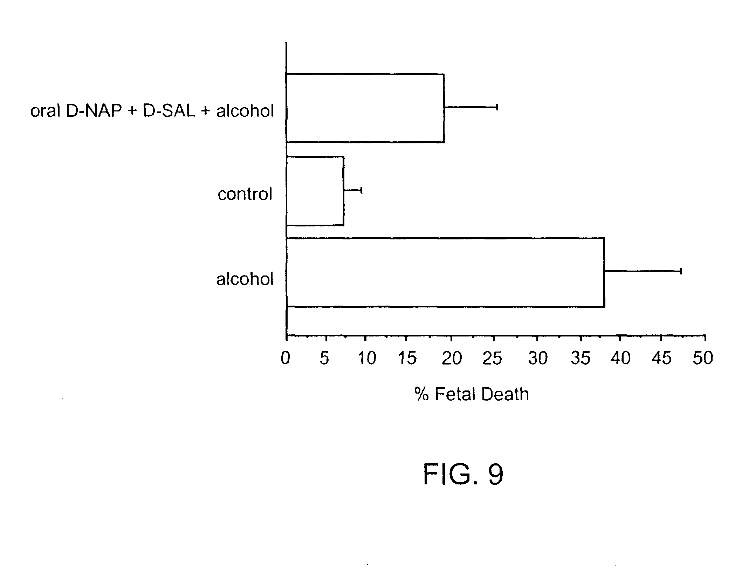

Figure 9 illustrates that oral treatment with D-NAP and D-SAL prevented fetal death associated with fetal alcohol syndrome. D-NAP and D-SAL were given by gavage immediately after alcohol treatment on E8. Comparisons are made to the alcohol group, overall ANOVA p=0.004. Post hoc Fishers tests were performed, with the oral treatment and control groups significantly different than alcohol (p<0.001 and ≤0.04 respectively). The sample sizes were control (21), alcohol (18), oral D-NAP + D-SAL and alcohol (18).

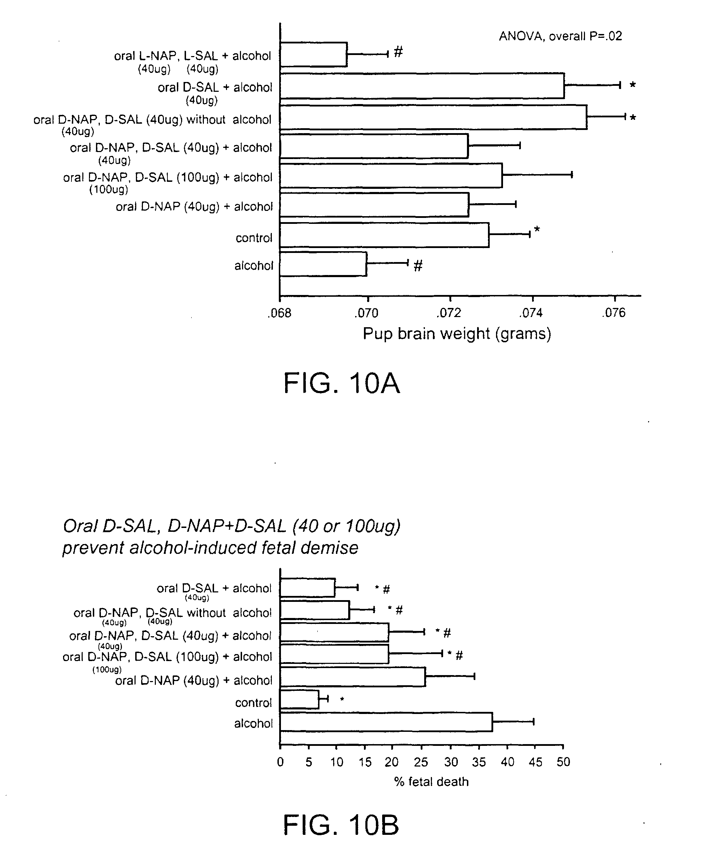

Figure 10A illustrates the effects of oral administration of ADNF polypeptides on pup brain weight. Pregnant mice were injected with alcohol as a model for fetal alcohol syndrome according to methods of Webster *et al*., *Neurobehav. Tox.* 2:227-34 (1980). The pregnant mice were injected 25% alcohol at 0.030 ml/g body weight. Peptide was dissolved in phosphate-buffered saline and administered orally by gavage 30 minutes prior to alcohol treatment. The dosage of peptides NAP and SAL administered to each mouse is shown in the figure. Error bars are ± 1 standard errors; * notes significant versus alcohol; and # notes significant versus control.
Figure 10B illustrates the effects of oral administration of ADNF polypeptides on fetal death. Pregnant mice were treated as described above in the description for Figure 10A. The dosage of peptides NAP and SAL administered to each mouse is shown in the figure. Error bars are ± 1 standard errors; * notes significant difference versus alcohol; and # notes significant difference versus control.

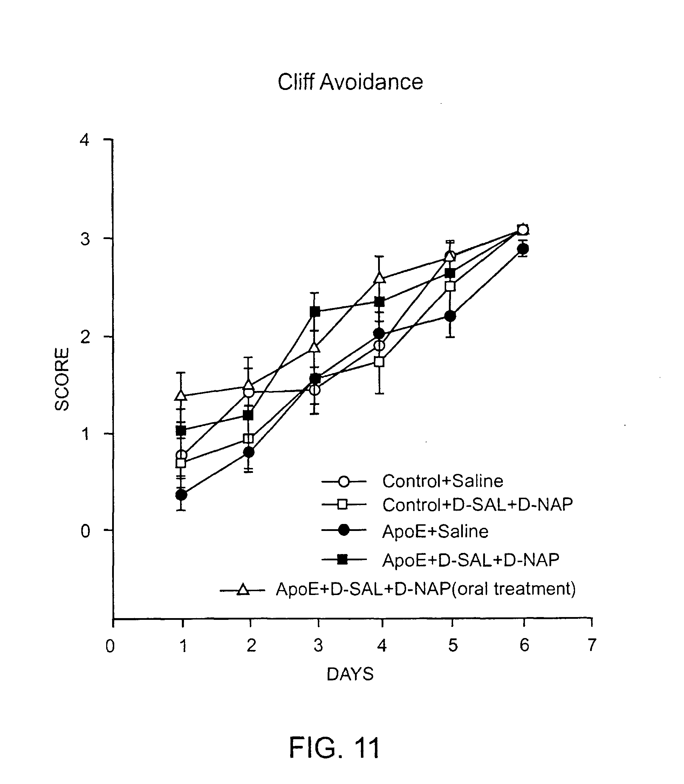

Figure 11 illustrates development of cliff avoidance behavior in newborn mice: comparison of peptide drug response in control vs. Apo-E knock-out mice. Animals were treated either by oral application, or subcutaneous injection of D-SAL + D-NAP. Peptides (0.5 mg each) were dissolved in 0.01M acetic acid (30 µl) and 470 µl saline. Further dilutions were performed in saline. For both applications, 0.5 µg of each of the test drugs were delivered; for the oral application (sublingual), in 10 µl saline and for the injection in 20 µl. This protocol was used for the first 4 days of life. From day 5-10, the amount of the peptides and the solution volume was doubled. From day 11-14, the amount of peptide was 2 µg each in 40 µl (oral) and 80 µl (injection). Tests performed daily included cliff avoidance, negative geotaxis, placing and righting behaviors. Both subcutaneous and oral administration of D-NAP and D-SAL were compared.

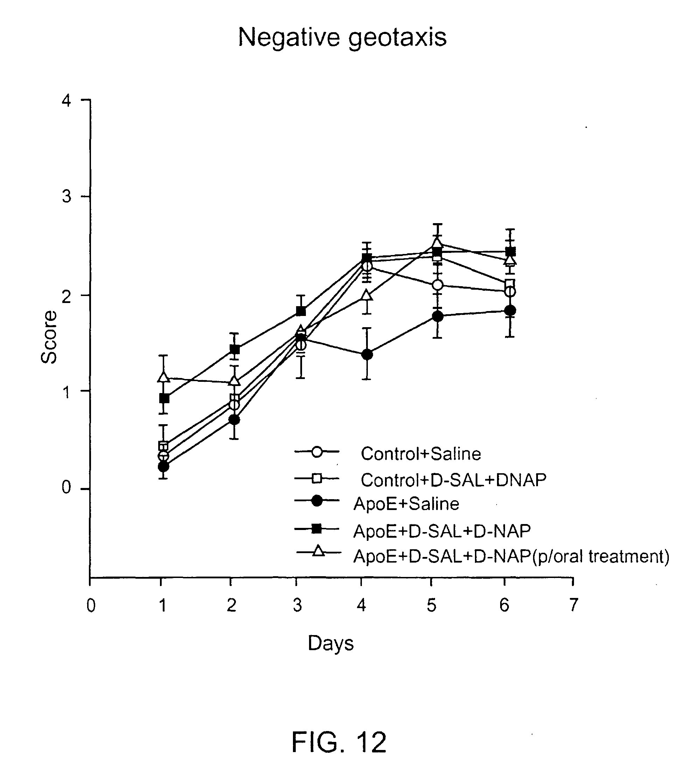

Figure 12 illustrates development of negative geotaxis behavior in newborn mice: comparison of peptide drug responses in control vs. Apo-E knock-out mice. Treatment paradigm was as described in Figure 11.

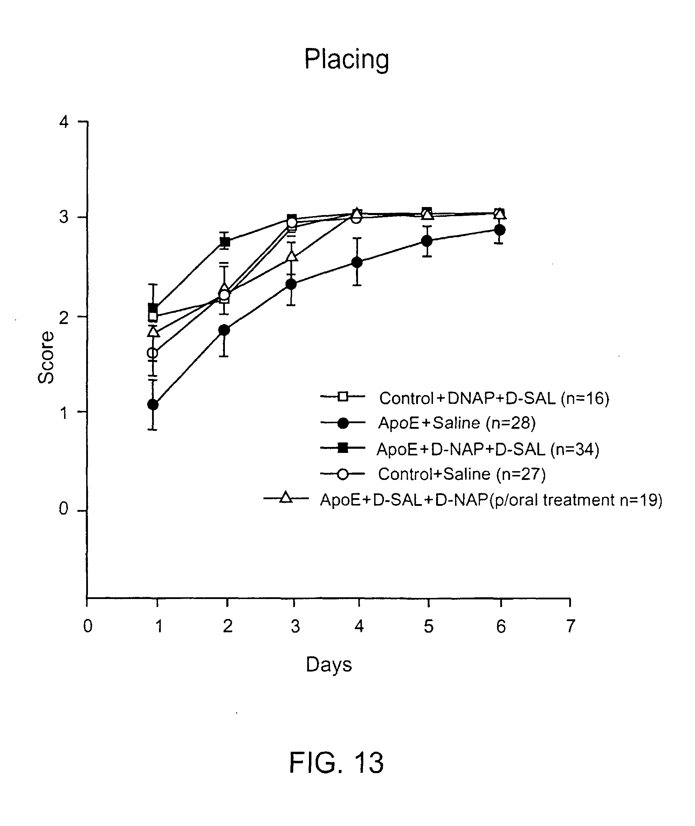

Figure 13 illustrates development of placing behavior in newborn mice: comparison of peptide drug responses in control vs. Apo-E knock-out mice. Treatment paradigm was as described in Figure 11.

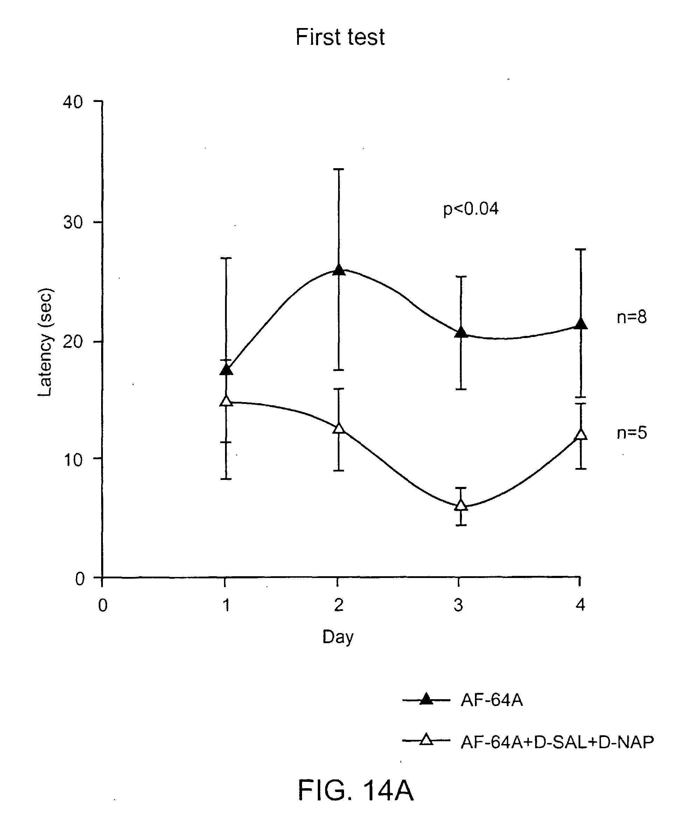

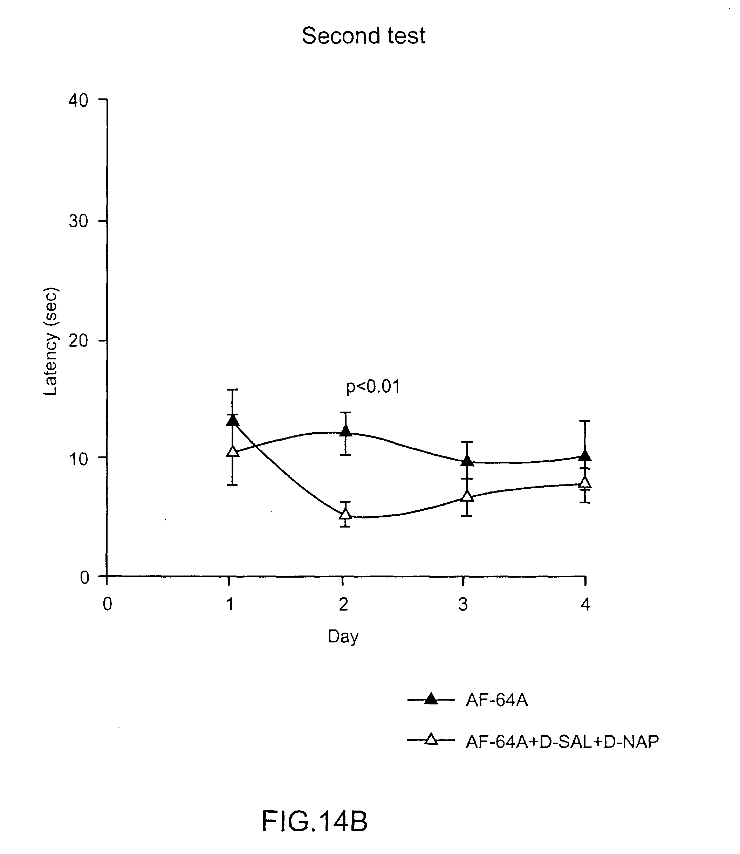

Figures 14 A and B illustrate the effect of oral administration of D-NAPVSIPQ + D-SALLRSIPA on learning and memory in rats treated with the cholinotoxin AF-64A. Short-term memory processes were examined by performance in the Morris water maze, measuring the time required to find the hidden platform in the second of two daily trials. The platform location and the starting point in which the animal was placed in the water were held constant within each pair of daily trials, but both locations were changed every day. For the first test, both the platform and the animal were situated in a new location with regard to the pool (with the pool being immobile). The experiment was performed as follows: the animal was positioned on the platform for 0.5 minute, then placed in the water. As shown in Figure 14A, the time required to reach the platform (indicative of learning and intact reference memory) was measured (first test). After 0.5 minute on the platform, the animal was placed back in the water (in the previous position) for an additional second test (Figure 14B) and search for the hidden platform (retained in the previous position). The time required to reach the platform in the second trial was recorded, indicative of short-term (working) memory. All measurements were performed using the computerized video-assisted HVS water maze system (HVS Image Ltd. Hampton, UK). Animals were tested for four days to eliminate random memory defective animals. The designated n is the number of animals tested. Each point is the mean + the standard error.

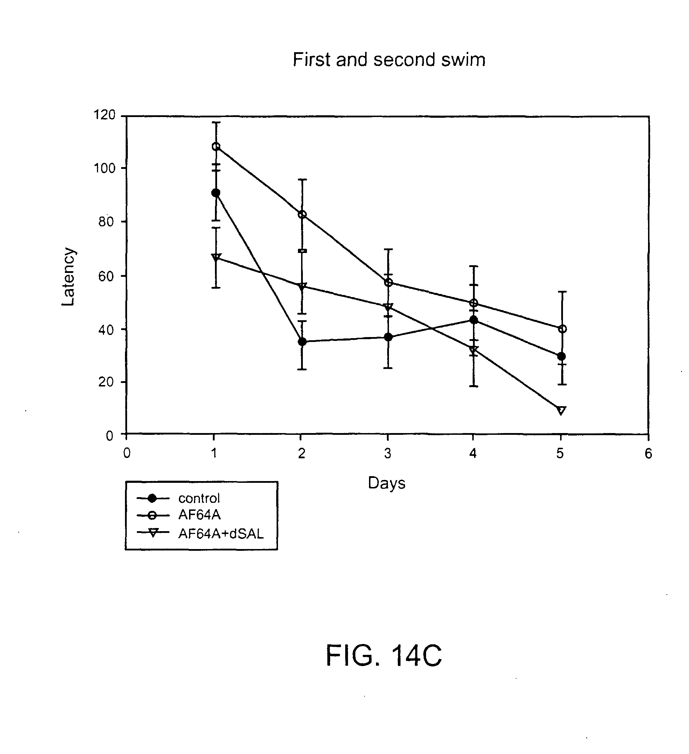

Figure 14C illustrates the effect of oral administration of D-SALLRSIPA alone on learning and memory in rats treated with the cholinotoxin AF-64A.

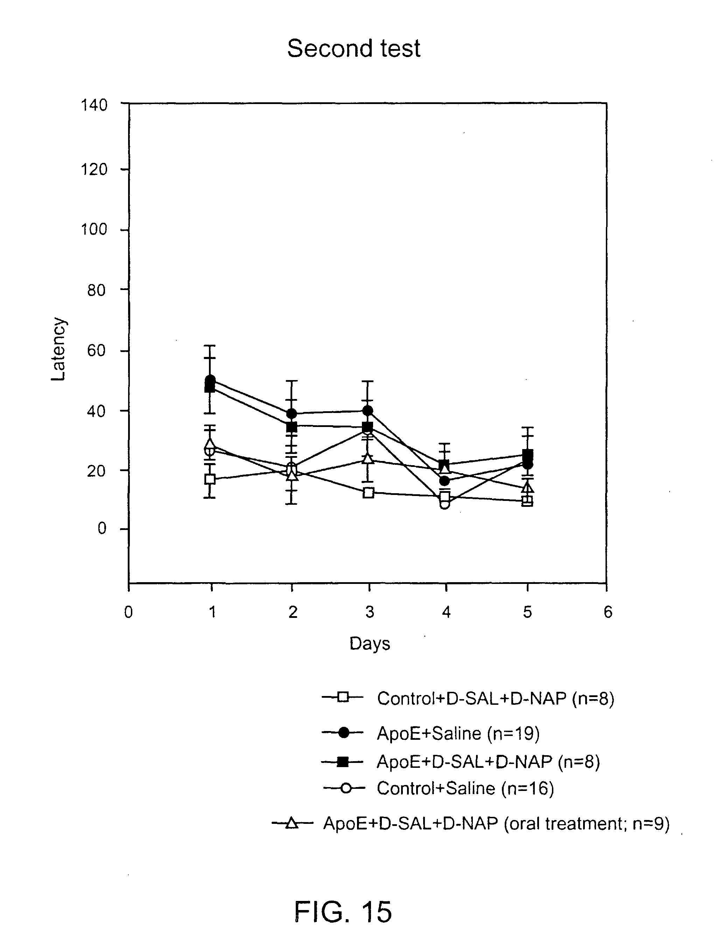

Figure 15 illustrates comparison of sublingual (oral) and subcutaneous administration of D-SAL + D-NAP in control vs. Apo-E knock-out mice assessed for short-term memory in the Morris swim maze. Improvements of cognitive functions were observed a week after cessation of the 2-week daily D-SAL + D-NAP treatment, *i*.*e*., in 21-day-old mice exposed to a 5-day training protocol. The time required to find the hidden platform in the second of two daily trials was measured. The platform location and the starting point in which the animal was placed in the water were held constant within each pair of daily trials, but both locations were changed every day. On the second test of the first trial day, the ApoE-deficient mice were significantly retarded as compared to controls (P<0.04) and improved after oral application of D-SAL + D-NAP, with most of the treated animals finding the platform at a latency of ≤ 20 sec.

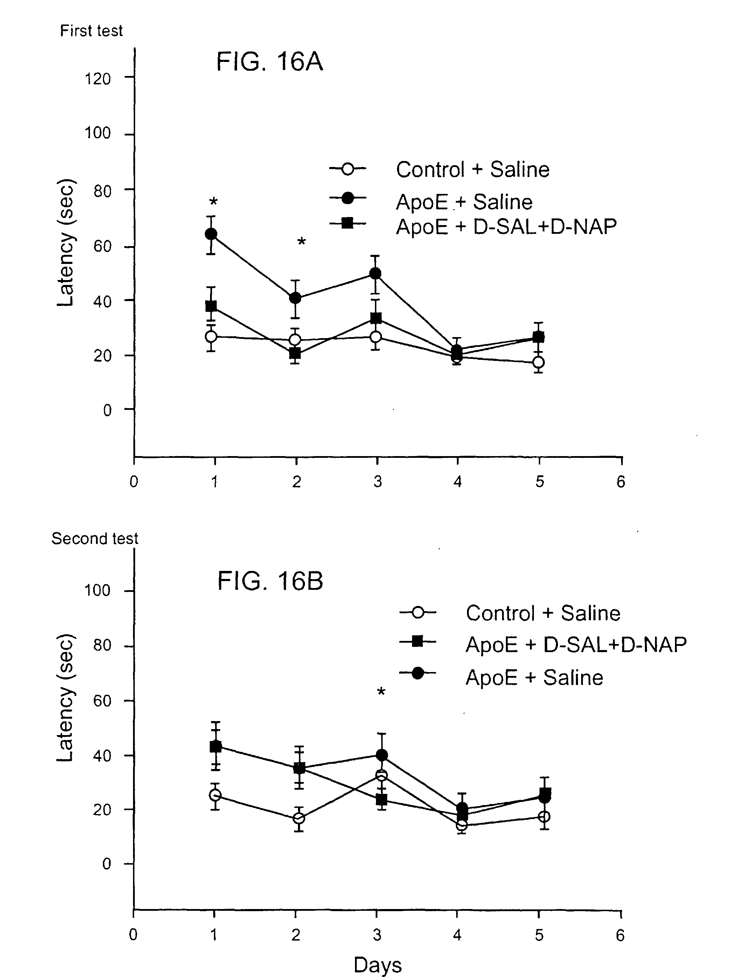

Figures 16A and 16B illustrate the first test and second test, respectively, of Morris water maze test results in apolipoprotein E-deficient mice. Experiments were performed following injections of a mixture of D-NAP + D-SAL with an injection protocol and Morris water maze as described in Gozes et al., *J*. *Pharmacol*. *Exp. Therap.* 293: 1091-1098 (2000). Results showed significant improvements on day 1 and day 2 (first daily test), and on day three (second daily test)- P<0.05.

### DEFINITIONS

The phrase "ADNF polypeptide" refers to one or more activity dependent neurotrophic factors (ADNF) that have an active core site comprising the amino acid sequence of SALLRSIPA or NAPVSIPQ, or conservatively modified variants thereof that have neurotrophic/neuroprotective activity as measured with *in vitro* cortical neuron culture assays described by, *e.g.*, Brenneman *et al., J*. *Pharmacol. Exp. Therp.* 285:629-627 (1998); Bassan *et al., J*. *Neurochem.* 72:1283-1293 (1999). An ADNF polypeptide can be an ADNF I polypeptide or an ADNF III polypeptide. An "ADNF polypeptide" can also refer to a mixture of ADNF I polypeptide and ADNF III polypeptide.

The term "ADNF I" refers to an activity dependent neurotrophic factor polypeptide having a molecular weight of about 14,000 Daltons with a pI of 8.3 ± 0.25. As described above, ADNF I polypeptides have an active core site comprising an amino acid sequence of Ser-Ala-Leu-Leu-Arg-Ser-Ile-Pro-Ala (also referred to as "SALLRSIPA," "SAL," or "ADNF I-9"; SEQ ID NO:1). *See,* Brenneman *et al., J*. *Clin. Invest.* 97:2299-2307 (1996), Glazner *et al., Anat. Embryol*. 200:65-71 (1999), Brenneman *et al., J*. *Pharm. Exp. Ther.* 285:619-27 (1998), Gozes & Brenneman, *J*. *Mol. Neurosci.* 7:235-244 (1996), and Gozes *et al., Dev. Brain Res.* 99:167-175 (1997), all of which are herein incorporated by reference. Unless indicated as otherwise, "SAL" refers to a peptide having an amino acid sequence of Ser-Ala-Leu-Leu-Arg-Ser-Ile-Pro-Ala (SEQ ID NO:1), not a peptide having an amino acid sequence of Ser-Ala-Leu.

The terms "ADNF III" and "ADNP" refer to an activity dependent neurotrophic factor polypeptide having a predicted molecular weight of about 95 kDa (about 828 amino acid residues) and a pI of about 5.99. As described above, ADNF III polypeptides have an active core site comprising an amino acid sequence of Asn-Ala-Pro-Val-Ser-Ile-Pro-Gln (also referred to as "NAPVSIPQ," "NAP," or "ADNF III-8"; SEQ ID NO:2). *See,* Bassan *et al*., *J*. *Neurochem.* 72:1283-1293 (1999), incorporated herein by reference. Unless indicated as otherwise, "NAP" refers to a peptide having an amino acid sequence of Asn-Ala-Pro-Val-Ser-Ile-Pro-Gln (SEQ ID NO:2), not a peptide having an amino acid sequence of Asn-Ala-Pro.

The phrase "reducing neuronal cell death" refers to reduction, including prevention, of neuronal cell death. Reduction is a change of a parameter by about 10% to about 100%, preferably at least about 50%, and more preferably at least about 80% compared to that of the control (*e.g.*, without treatment with, *e.g.*, ADNF polypeptides). The reduction of neuronal cell death can be measured by any methods known in the art. For example, ADNF polypeptides that reduce neuronal cell death can be screened using the various methods described in U.S.S.N. 60/037,404, filed February 27, 1997 (published as WO98/35042) and U.S.S.N. 09/187,330, filed November 6, 1998.

The phrase "oxidative stress" in cells or tissues refers to enhanced generation of free radicals or reactive oxygen species (ROS) (such as α-hydroxy ethyl radical, superoxide radical, hydroxy radical, peroxy radical, and hydrogen peroxide) and/or a depletion in antioxidant defense system causing an imbalance between prooxidants and antioxidants. Enzymatic antioxidant system includes, *e.g.*, superoxide dismutase, catalase, glutathione peroxidase, and glutathione reductase, and nonenzymatic antioxidants include, *e*.*g*., reduced glutathione, vitamin A, C, and E. *See,* Schlorff *et al*., *Alcohol* 17:97-105 (1999).

The phrase "reducing oxidative stress" refers to reduction, including prevention, of oxidative stress in cells and tissues. Reduction is a change of a parameter by about 10% to about 100%, preferably at least about 50%, and more preferably at least about 80% compared to that of the control (*e.g.*, without treatment with, *e.g*., ADNF polypeptides). The reduction in oxidative stress can be measured by any methods known in the art. For example, ADNF polypeptides that reduce oxidative stress can be screened by using primary neurons treated with FeSO₄ *in vitro* as described *infra.* Also, ADNF polypeptides that reduce oxidative stress can be screened using animals that ingested ethanol which is known to cause oxidative stress in cells and tissues. For example, the effects of ADNF polypeptides on lipid peroxidation in plasma and/or antioxidant system of rats that ingested ethanol can be used. *See, e.g.,* Schlorff *et al*., *Alcohol* 17:97-105 (1999).

The phrases "fetal alcohol syndrome" and "fetal alcohol effects" relate to various physical and mental conditions of an embryo, a fetus, or a subject who is exposed to alcohol *in utero* (*e.g.,* whose mother consumed alcohol during pregnancy) in an amount sufficient to initiate the development of these conditions or to cause these conditions in the absence of prevention treatment, *e.g.*, treatment with ADNF polypeptides. Some of these conditions include, but are not limited to, the following:
skeletal deformities: deformed ribs and sternum; curved spine; hip dislocations; bent, fused, webbed, or missing fingers or toes; limited movement of joints; small head; facial abnormalities: small eye openings; skin webbing between eyes and base of nose; drooping eyelids; nearsightedness; failure of eyes to move in same direction; short upturned nose; sunken nasal bridge; flat or absent groove between nose and upper lip; thin upper lip; opening in roof of mouth; small jaw; low-set or poorly formed ears; organ deformities: heart defects; heart murmurs; genital malformations; kidney and urinary defects; central nervous system handicaps: small brain; faulty arrangement of brain cells and connective tissue; mental retardation - usually mild to moderate, but occasionally severe; learning disabilities; short attention span; irritability in infancy; hyperactivity in childhood; poor body, hand, and finger coordination (*see, e.g.,* www.well.com/user/woa/fsfas.htm); and other abnormalities: brain weight reduction, body weight reduction, a higher rate of death *in utero,* and a decrease in the level of VIP (*e.g.*, VIP mRNA).

The phrase "reducing a condition associated with fetal alcohol syndrome" refers to reduction, including prevention, of parameters associated with fetal alcohol syndrome. Reduction is a change of a parameter by about 10% to about 100%, preferably at least about 50%, and more preferably at least about 80% compared to that of the control (*e*.*g*., exposed to alcohol *in utero* without any treatment, *e*.*g*., treatment with ADNF polypeptides). The parameters can be any physical or mental condition listed above. For example, they can be: (1) the percentage of fetus death, (2) fetal weights and fetal brain weights, (3) the level of VIP (*e.g.*, VIP mRNA) in embryos, (4) learning and/or memory, and (5) the glutathione level.

The phrase "a subject with fetal alcohol syndrome" relates to an embryo, a fetus, or a subject, in particular a human, who is exposed to alcohol *in utero* and who has fetal alcohol syndrome or who is at risk or in danger of developing, due to maternal alcohol consumption, any of the conditions related to fetal alcohol syndrome, such as the effects described above.

The term "memory" includes all medical classifications of memory, *e.g.*, sensory, immediate, recent and remote, as well as terms used in psychology, such as reference memory, which refers to information gained from previous experience, either recent or remote (*see, e.g, Harrison's Principles of Internal Medicine,* volume 1, pp. 142-150 (Fauci *et al*., eds., 1988)).

The term "contacting" is used herein interchangeably with the following: combined with, added to, mixed with, passed over, incubated with, or flowed over. Moreover, the ADNF polypeptides of the present invention can be "administered" by any conventional method such as, for example, parenteral, oral, topical, and inhalation routes. In preferred embodiments, oral administration is employed. In the context of methods related to fetal alcohol syndrome, ADNF polypeptides can be administered directly to an embryo, a fetus, or a subject *in utero* or to the subject *in utero* indirectly, by administering the polypeptide to the mother by any other methods described herein.

"An amount sufficient" or "an effective amount" is that amount of a given ADNF polypeptide that reduces neuronal cell death or reduces fetal alcohol syndrome or oxidative stress as described herein. For example, in the context of neuronal death, "an amount sufficient" or "an effective amount" is that amount of a given ADNF polypeptide that reduces neuronal cell death in the assays of, *e.g.,* Hill *et al*., *Brain Res.* 603:222-233 (1993); Brenneman *et al., Nature* 335:639-642 (1988); or Brenneman *et al., Dev. Brain Res.* 51:63-68 (1990); Forsythe & Westbrook, *J. Physiol. Lond.* 396:515-533 (1988). In the context of reducing oxidative stress, "an amount sufficient" or "an effective amount" is that amount of ADNF polypeptide that reduces or prevents, *e.g.*, changes in lipid peroxidation in plasma or changes in antioxidant system in accordance with the assays described in Schlorff *et al*., *Alcohol* 17:97-105 (1999). In the context of reducing fetal alcohol syndrome, "an amount sufficient" or "an effective amount" is that amount of a given ADNF polypeptide that reduces or prevents, for example, (1) the percentage of fetus death, (2) a reduction in fetal weights and fetal brain weights, or (3) a reduction in the level of VIP mRNA in embryos. The dosing range can vary depending on the ADNF polypeptide used, the route of administration and the potency of the particular ADNF polypeptide, but can readily be determined using the foregoing assays.

The terms "isolated," "purified," or "biologically pure" refer to material that is substantially or essentially free from components which normally accompany it as found in its native state. Purity and homogeneity are typically determined using analytical chemistry techniques such as polyacrylamide gel electrophoresis or high performance liquid chromatography. A protein that is the predominant species present in a preparation is substantially purified. In particular, an isolated ADNF nucleic acid is separated from open reading frames that flank the ADNF gene and encode proteins other than ADNF. The term "purified" denotes that a nucleic acid or protein gives rise to essentially one band in an electrophoretic gel. Particularly, it means that the nucleic acid or protein is at least 85% pure, more preferably at least 95% pure, and most preferably at least 99% pure.

"Nucleic acid" refers to deoxyribonucleotides or ribonucleotides and polymers thereof in either single- or double-stranded form. The term nucleic acid is used interchangeably with gene, cDNA, mRNA, oligonucleotide, and polynucleotide.

The term "amino acid" refers to naturally occurring amino acids in L-form and their enantiomers in D-form. The two-mirror-image forms (enantiomers) of amino acids are called the L-isomer and the D-isomer, where L refers to levorotatory (left rotation of the plane of polarization of light) and D refers to dextrorotatory (right rotation of the plane of polarization).

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

The terms "polypeptide," "peptide," and "protein" are used interchangeably herein to refer to a polymer of amino acid residues. The terms apply to amino acid polymers in which one or more amino acid residue is a naturally occurring amino acid in L-form or their enantiomers in D-form or combinations thereof.

### DETAILED DESCRIPTION OF THE INVENTION AND PREFERRED EMBODIMENTS

### I. INTRODUCTION

The chirality (left or right handedness) of a peptide pertains to the tetrahedral array of four different groups about the α-carbon atom of the constituent amino acids that confers optical activity. The two-mirror-image forms (enantiomers) of amino acids are called the L-isomer and the D-isomer, where L refers to levorotatory (left rotation of the plane of polarization of light) and D refers to dextrorotatory (right rotation of the plane of polarization). Only L-amino acids are constituents of naturally occurring proteins. Classical receptor pharmacology teaches that membrane receptors readily discriminate between L-and D-agonists and antagonists. Receptor activation is mediated through a stereoselective preference for agents in the naturally occurring L-isomer form.

Because ADNF I and ADNF III polypeptides are neurotrophic factors, it was predicted that ADNF I and ADNF III polypeptides comprising D-amino acids would not be able to activate their respective stereoselective membrane receptors. Surprisingly, it was found that ADNF I and ADNF III polypeptides comprising D-amino acids were bioactive. In fact, all D- and all L-amino acid forms of the active core site peptide from ADNF I polypeptides, *i*.*e*., SALLRSIPA (SAL), were virtually identical in neuronal survival activity *in vitro.* Similarly, all D- and all L-amino acid forms of the active core site peptide from ADNF III polypeptides, *i*.*e*., NAPVSIPQ (NAP), were virtually identical in neuronal survival activity *in vitro.* It is very uncommon that all D-amino acid peptides are active, and even more uncommon that the D- and L-isomers of a given peptide are equally active.

A few examples of peptides with similar actions in D- vs. L-forms have been reported. A well-known example is beta amyloid. It was shown that bioactivity of D-isomers of beta amyloid 1-42 is identical to that observed with the L-form of the peptide (Cribbs *et al*., *J*. *Biol*. *Chem*. 272:7431-7436 (1997)). Another example is the immunosuppressive effects of D- and L-peptides derived from the HLA class I heavy chain (Woo *et al*., *Transplantation* 64:1460-1467 (1997)). Although these examples illustrate that bioactivity of peptides can be non-stereoselective, this phenomenon is very rare. This is because biological macromolecules are made up of monomer molecules of uniform chirality (Mason, *Chirality* 3:223 (1990)) and the biochemical interactions of biological macromolecules are inherently chiral. In fact, for neurotrophic agents, there is no known example that exhibits non-chiral properties. Thus, it is surprising that ADNF polypeptides of the present invention provide neuroprotection through a non-chiral mechanism.

The fact that ADNF polypeptides comprising D-amino acids are bioactive allows these polypeptides to be administered orally. Compared to L-isomers, D-isomers of peptides have increased stability in the gastrointestinal tract and can be absorbed without change (He *et al*., *J*. *Pharmaceutical Sci.* 87:626-633 (1998)). For example, in He *et al*., bioavailability (as measured by the appearance of unchanged labeled D-peptides in the urine after 24-48 hours) was estimated at 13% with compounds of molecular mass of 900 Daltons, the approximate size of the active core sites of ADNF I and ADNF III polypeptides. ADNF polypeptides comprising D-amino acids provide a longer bioavailability, and thus can be formulated for oral administration.

As such, the present invention provides for the first time ADNF polypeptides comprising at least one D-amino acid within their active core sites, preferably at the N-terminus and/or the C-terminus of the active core sites. In a presently preferred embodiment, the invention provides ADNF polypeptides comprising all D-amino acids. The invention also provides a pharmaceutical composition comprising a pharmaceutically acceptable excipient and an ADNF I polypeptide, an ADNF III polypeptide, or a mixture thereof, wherein at least one of the ADNF I polypeptide or the ADNF III polypeptide comprises at least one D-amino acid within its active core site. In particular, the invention provides an orally active pharmaceutical composition comprising an ADNF polypeptide comprising at least one D-amino acid within its active core site. The ADNF polypeptides and the pharmaceutical compositions of the present invention can be used, for the manufacture of a medicament for reducing neuronal cell death, for reducing oxidative stress in a patient, and for reducing a condition associated with fetal alcohol syndrome.

### II. ADNF POLYPEPTIDES COMPRISING D-AMINO ACIDS AND METHODS OF MAKING THE POLYPEPTIDES

In one aspect, the present invention provides an ADNF polypeptide comprising at least one D-amino acid within its active core site, preferably at the N-terminus and/or the C-terminus of the active core site. Since D-enantiomers of polypeptides are enzymatically more stable than their L-enantiomers, an ADNF polypeptide comprising D-amino acids provides a longer bioavailability compared to its counterpart comprising L-amino acids. In particular, the ADNF polypeptides comprising D-amino acids are stable in the gastrointestinal tract and can be absorbed without cleavage in the human body. Therefore, the ADNF polypeptides of the present invention are particularly useful as an oral agent.

In one embodiment, the ADNF I polypeptide comprises an active core site having the following amino acid sequence: Ser-Ala-Leu-Leu-Arg-Ser-Ile-Pro-Ala (SEQ ID NO:1), wherein the active core site comprises at least one D-amino acid. In another embodiment, both the N-terminal and/or the C-terminal amino acids of the active core site of the ADNF I polypeptide are D-amino acids. In yet another embodiment, the active core site of the ADNF I polypeptide comprises D-amino acids at locations other than at the N-terminus and/or the C-terminus of the active core site. For example, any amino acids within the active core site can be a D-amino acid. In other words, any one or any combinations of serine, alanine, leucine, leucine, arginine, serine, isoleucine, proline, and alanine within the active core site of the ADNF I polypeptides can be a D-amino acid. For instance, every other amino acid within the active core site of the ADNF I polypeptide can be a D-amino acid. In a preferred embodiment, the active core site of the ADNF I polypeptide comprises all D-amino acids.

In yet another embodiment, the ADNF I polypeptide can comprise additional amino acids at the N-terminus and/or at the C-terminus of the active core site. For example, the ADNF I polypeptide can comprise up to 20 amino acids at the N-terminus and/or the C-terminus of the active core site. In these embodiments, preferably the N-terminal amino acid and/or the C-terminal amino acid of the ADNF I polypeptide are D-amino acids. Any one of the additional amino acids or all of the additional amino acids can be D-amino acids. In a preferred embodiment, the ADNF I polypeptide does not comprise any additional amino acids and has an amino acid sequence of Ser-Ala-Leu-Leu-Arg-Ser-Ile-Pro-Ala (SEQ ID NO:1), wherein the ADNF I polypeptide comprises all D-amino acids.

In another embodiment, the ADNF III polypeptide comprises an active core site having the following amino acid sequence: Asn-Ala-Pro-Val-Ser-Ile-Pro-Gln (SEQ ID NO:2), wherein the active core site comprises at least one D-amino acid. In another embodiment, both the N-terminal and/or C-terminal amino acids of the active core site of the ADNF III polypeptide are D-amino acids. In yet another embodiment, the active-core site of the ADNF III polypeptide comprises D-amino acids at locations other than at the N- or C-terminus of the active core site. For example, any amino acids within the active core site can be a D-amino acid. In other words, any one or any combination of asparagine, alanine, proline, valine, serine, isoleucine, proline, and glutamine within the active core site of the ADNF III polypeptides can be a D-amino acid. For instance, every other amino acid within the active core site of the ADNF III polypeptide can be a D-amino acid. In a preferred embodiment, the active core site of the ADNF III polypeptide comprises all D-amino acids.

In yet another embodiment, the ADNF III polypeptide can comprise additional amino acids at the N-terminus and/or at the C-terminus of the active core site. For example, the ADNF III polypeptide can comprise up to 40 amino acids at the N-terminus and/or the C-terminus of the active core site. In another example, the ADNF III polypeptide can comprise up to 20 amino acids at the N-terminus and/or the C-terminus of the active core site. In yet another example, the ADNF III polypeptide can comprise up to 10 amino acids at the N-terminus and/or the C-terminus of the active core site. In these embodiments, preferably the N-terminal amino acid and/or the C-terminal amino acid of the ADNF III polypeptide are D-amino acids. Any one of the additional amino acids or all of the additional amino acids can be D-amino acids. In a preferred embodiment, the ADNF III polypeptide does not comprise any additional amino acids and has an amino acid sequence of Asn-Ala-Pro-Val-Ser-Ile-Pro-Gln (SEQ ID NO:2), wherein the ADNF III polypeptide comprises all D-amino acids.

In a preferred embodiment, the ADNF I polypeptide comprises an amino acid sequence having the following formula: (R¹)ₓ-Ser-Ala-Leu-Leu-Arg-Ser-Ile-Pro-Ala-(R²)_{y} (SEQ ID NO:3), wherein the active core site comprises at least one D-amino acid. In another preferred embodiment, the ADNF III polypeptide comprises an amino acid sequence having the following formula: (R³)_{w}-Asn-Ala-Pro-Val-Ser-Ile-Pro-Gln-(R⁴)_{z} (SEQ ID NO:4), wherein the active core site comprises at least one D-amino acid. In these preferred embodiments, the previous discussion pertaining to the location and the number of D-amino acids within the active core sites of the ADNF I and ADNF III polypeptides is fully applicable, and thus, will not be repeated with respect to these particular embodiments of the invention.

In the above formula, each of R¹, R², R³, and R⁴, if present, is an amino acid sequence comprising from 1 to about 40 amino acids wherein each amino acid in the amino acid sequence is independently selected. The term "independently selected" is used herein to indicate that the amino acids making up, for example, the amino acid sequence R¹ may be identical or different (*e.g.,* all of the amino acids in the amino acid sequence may be threonine). This discussion pertaining to R¹ is fully applicable to R², R³, and R⁴. Moreover, any one or any combinations of the amino acids making up the amino acid sequence R¹ can be a D-amino acid or an L-amino acid. In one embodiment, the N-terminal amino acid of R¹ is a D-amino acid and/or the C-terminal amino acid of R² is a D-amino acid. In another embodiment, the N-terminal amino acid of R³ is a D-amino acid and/or the C-terminal amino acid of R⁴ is a D-amino acid. In another embodiment, each of R¹, R², R³, and R⁴ comprises all D-amino acids.

Within the above formula for the ADNF I polypeptide, x and y are independently selected and are equal to zero or one. The term independently selected is used herein to indicate that x and y may be identical or different. For example, x and y may both be zero or, alternatively, x and y may both be one. In addition, x may be zero and y may be one or, alternatively, x may be one and y may be zero. Moreover, if x and y are both one, the amino acid sequences R¹ and R² may be the same or different. As such, the amino acid sequences R¹ and R² are independently selected. If R¹ and R² are the same, they are identical in terms of both chain length and amino acid composition. For example, both R¹ and R² may be Val-Leu-Gly-Gly-Gly (SEQ ID NO:5). If R¹ and R² are different, they can differ from one another in terms of chain length and/or amino acid composition and/or order of amino acids in the amino acids sequences. For example, R¹ may be Val-Leu-Gly-Gly-Gly (SEQ ID NO:5), whereas R² may be Val-Leu-Gly-Gly (SEQ ID NO:9). Alternatively, R¹ may be Val-Leu-Gly-Gly-Gly (SEQ ID NO:5), whereas R² may be Val-Leu-Gly-Gly-Val (SEQ ID NO:13). Alternatively, R¹ may be Val-Leu-Gly-Gly-Gly (SEQ ID NO:5), whereas R² may be Gly-Val-Leu-Gly-Gly (SEQ ID NO:11).

Similarly, w and z are independently selected and are equal to zero or one within the above formula for the ADNF III polypeptide. The term independently selected is used herein to indicate that w and z may be identical or different. For example, w and z may both be zero or, alternatively, w and z may both be one. In addition, w may be zero and z may be one or, alternatively, w may be one and z may be zero. Moreover, if w and z are both one, the amino acid sequences R³ and R⁴ may be the same or different. As such, the amino acid sequences R³ and R⁴ are independently selected. If R³ and R⁴ are the same, they are identical in terms of both chain length and amino acid composition. For example, both R³ and R⁴ may be Leu-Gly-Leu-Gly-Gly (SEQ ID NO:7). If R³ and R⁴ are different, they can differ from one another in terms of chain length and/or amino acid composition and/or order of amino acids in the amino acids sequences. For example, R³ may be Leu-Gly-Leu-Gly-Gly (SEQ ID NO:7), whereas R⁴ may be Leu-Gly-Leu-Gly (SEQ ID NO:12). Alternatively, R³ may be Leu-Gly-Leu-Gly-Gly (SEQ ID NO:7), whereas R⁴ may be Leu-Gly-Leu-Gly-Leu (SEQ ID NO:13).

Within the scope, certain ADNF I and ADNF III polypeptides are preferred, namely those in which x, y, w, and z are all zero (*i.e.*, SALLRSIPA (SEQ ID NO:1) and NAPVSIPQ (SEQ ID NO:2), respectively). Equally preferred are ADNF I polypeptides in which x is one; R¹ is Val-Leu-Gly-Gly-Gly (SEQ ID NO:5); and y is zero. Also equally preferred are ADNF I polypeptides in which x is one; R¹ is Val-Glu-Glu-Gly-Ile-Val-Leu-Gly-Gly-Gly (SEQ ID NO:6); and y is zero. Also equally preferred are ADNF III polypeptides in which w is one; R³ is Gly-Gly; and z is zero. Also equally preferred are ADNF III polypeptides in which w is one; R³ is Leu-Glu-Gly; z is one; and R⁴ is Gln-Ser. Also equally preferred are ADNF III polypeptides in which w is one; R³ is Leu-Gly-Leu-Gly-Gly- (SEQ ID NO:7); z is one; and R⁴ is Gln-Ser. Also equally preferred are ADNF III polypeptides in which w is one; R³ is Ser-Val-Arg-Leu-Gly-Leu-Gly-Gly (SEQ ID NO:8); z is one; and R⁴ is Gln-Ser. Additional amino acids can be added to both the N-terminus and the C-terminus of these active core sites (SALLRSIPA or NAPVSIPQ) without loss of biological activity as evidenced by the fact that the intact ADNF I or ADNF III growth factors exhibit extraordinary biological activity. *See,* U.S.S.N. 08/324,297, filed October 17, 1994 (also published as WO96/11948) for the description of ADNF I polypeptides; and U.S.S.N. 60/037,404 filed February 27, 1997 and U.S.S.N. 60/059,621 filed, September 23, 1997 (also published as WO98/35042) for the description of ADNF III polypeptides.

The ADNF polypeptides comprising at least one D-amino acid within the active core sites of the ADNF polypeptides can be prepared via a wide variety of well-known techniques. Polypeptides of relatively short size are typically synthesized in solution or on a solid support in accordance with conventional techniques (*see, e.g.,* Merrifield, *Am. Chem. Soc.* 85:2149-2154 (1963)). Various automatic synthesizers and sequencers are commercially available and can be used in accordance with known protocols (*see, e.g.,* Stewart & Young, *Solid Phase Peptide Synthesis* (2nd ed. 1984)). Solid phase synthesis in which the C-terminal amino acid of the sequence is attached to an insoluble support followed by sequential addition of the remaining amino acids in the sequence is the preferred method for the chemical synthesis of the polypeptides of this invention. Using solid phase synthesis methods, one or more D-amino acids can be inserted, instead of L-amino acids, into an ADNF polypeptide at any desired location(s). Techniques for solid phase synthesis are described by Barany & Merrifield, *Solid-Phase Peptide Synthesis;* pp. 3-284 in *The Peptides: Analysis, Synthesis, Biology. Vol. 2: Special Methods in Peptide Synthesis, Part A*.; Merrifield *et al*., *J*. *Am. Chem. Soc.* 85:2149-2156 (1963); and Stewart *et al*., *Solid Phase Peptide Synthesis* (2nd ed. 1984).

Alternatively, ADNF polypeptides comprising at least one D-amino acid within their active core sites can be synthesized using both recombinant DNA methods and chemical synthesis. For example, fragments of an ADNF polypeptide comprising D-amino acids can be chemically synthesized using solid phase synthesis methods described above, and fragments of an ADNF polypeptide comprising L-amino acids can be produced recombinantly. That is, expression vectors containing a nucleic acid encoding a fragment of an ADNF polypeptide can be introduced into host cells, and then the expressed ADNF polypeptide fragments can be purified. These ADNF polypeptide fragments comprising D-amino acids and ADNF polypeptide fragments comprising L-amino acids can then be chemically linked to one another.

After chemical synthesis, biological expression or purification, the polypeptide(s) may possess a conformation substantially different than the native conformations of the constituent polypeptides. In this case, it is helpful to denature and reduce the polypeptide and then to cause the polypeptide to re-fold into the preferred conformation. Methods of reducing and denaturing polypeptides and inducing re-folding are well known to those of skill in the art (*see* Debinski *et al., J*. *Biol. Chem.* 268:14065-14070 (1993); Kreitman & Pastan, *Bioconjug. Chem.* 4:581-585 (1993); and Buchner *et al*., *Anal. Biochem.* 205:263-270 (1992)). Debinski *et al*., for example, describe the denaturation and reduction of inclusion body polypeptides in guanidine-DTE. The polypeptide is then refolded in a redox buffer containing oxidized glutathione and L-arginine.

### III. PHARMACEUTICAL COMPOSITIONS

In another aspect, the present invention provides pharmaceutical compositions comprising at least one of the previously described ADNF polypeptides comprising at least one D-amino acid within the active core site in an amount sufficient to exhibit neuroprotective/neurotrophic activity, and a pharmaceutically acceptable diluent, carrier or excipient. The pharmaceutical compositions comprising one of the previously described ADNF polypeptides are particularly useful as oral agents, as they have stability in the gastrointestinal tract and can be absorbed without change. Moreover, by using mixtures of ADNF polypeptides in L-form and in D-form for producing pharmaceutical compositions, pharmaceutical compositions possessing varying dose response properties can be obtained. These pharmaceutical compositions are useful, *inter alia,* in targeting different receptors that may have different affinities for ADNF polypeptides, or to provide custom tailored drug treatment regime for individuals affected by, *e.g.*, neurodegenerative disorders.

In one embodiment, the pharmaceutical composition comprises a pharmaceutically acceptable excipient and an ADNF polypeptide, wherein the ADNF polypeptide is a member selected from the group consisting of: (a) an ADNF I polypeptide comprising an active core site having the following amino acid: Ser-Ala-Leu-Leu-Arg-Ser-Ile-Pro-Ala (SEQ ID NO:1); (b) an ADNF III polypeptide comprising an active core site having the following amino acid: Asn-Ala-Pro-Val-Ser-Ile-Pro-Gln (SEQ ID NO:2); and (c) a mixture of the ADNF I polypeptide or part (a) and the ADNF III polypeptide of part (b); wherein at least one of the ADNF I polypeptide and the ADNF III polypeptide comprises an active core site comprising at least one D-amino acid, preferably at the N-terminus and/or the C-terminus of the active core site.

In another embodiment, the pharmaceutical composition comprises a pharmaceutically acceptable excipient and an ADNF I polypeptide, wherein the active core site of the ADNF I polypeptide comprises at least one D-amino acid, preferably at the N-terminus and/or the C-terminus of the active core site. The previous discussion pertaining to the location and the number of D-amino acids within the active core site of ADNF I, as well as the discussion of additional D- and/or L-amino acids added on to the active site of the ADNF I polypeptide is fully applicable, and thus, will not be repeated with respect to this particular embodiment of the invention.

In yet another embodiment, the pharmaceutical composition comprises a pharmaceutically acceptable excipient and an ADNF III polypeptide, wherein the active core site of the ADNF III polypeptide comprises at least one D-amino acid, preferably at the N-terminus and/or the C-terminus of the active core site. The previous discussion pertaining to the location and the number of D-amino acids within the active core site of ADNF III, as well as the discussion of additional D- and/or L-amino acids added on to the active site of the ADNF III polypeptide is fully applicable, and thus, will not be repeated with respect to this particular embodiment of the invention.

In yet another embodiment, the pharmaceutical composition comprises a pharmaceutically acceptable excipient and a mixture of an ADNF I polypeptide and an ADNF III polypeptide, wherein at least one of the ADNF I and the ADNF III polypeptides comprises an active core site comprising at least one D-amino acid. The previous discussion pertaining to the location and the number of D-amino acids within the active core site of ADNF I or ADNF III, as well as the discussion of additional D- and/or L-amino acids added on to the active site of the ADNF I or ADNF III polypeptide is fully applicable, and thus, will not be repeated with respect to this particular embodiment of the invention. In yet another embodiment, the pharmaceutical composition comprises an ADNF I polypeptide comprising all D-amino acids and an ADNF III polypeptide comprising all L-amino acids. In yet another embodiment, the pharmaceutical composition comprises an ADNF I polypeptide comprising all L-amino acids and an ADNF III polypeptide comprising all D-amino acids. In yet another embodiment, the pharmaceutical composition comprises an ADNF I polypeptide comprising all D-amino acids and an ADNF III polypeptide comprising all D-amino acids.

In a pharmaceutical composition, any one or more of the ADNF I polypeptide described herein can be mixed with any one or more of the ADNF III polypeptide described herein. A mixture of an ADNF I polypeptide and an ADNF III polypeptide can be a blend of two or more of these polypeptides. A mixture of an ADNF I polypeptide and an ADNF III polypeptide can also refer to one or more of ADNF I polypeptides that are coupled to one or more of ADNF III polypeptides. For example, an ADNF I polypeptide can be covalently linked to an ADNF III polypeptide. A mixture of an ADNF I polypeptide and an ADNF III polypeptide can be prepared as a single composition and can be administered to a subject. Alternatively, an ADNF I polypeptide and an ADNF III polypeptide can be prepared as separate compositions and can be administered simultaneously or sequentially to a subject. Furthermore, different proportions of an ADNF I polypeptide and an ADNF III polypeptide can be administered to a subject. For example, in a mixture the ratio of an ADNF I polypeptide and an ADNF III polypeptide can be in the range of 1:100 to 100:1, 1:10 to 10:1, or 1:2 to 2:1.

The pharmaceutical compositions of the present invention are suitable for use in a variety of drug delivery systems. Suitable formulations for use in the present invention are found in *Remington's Pharmaceutical Sciences* (17th ed. 1985)), which is incorporated herein by reference. A brief review of methods for drug delivery is described in, *e.g.,* Langer, *Science* 249:1527-1533 (1990), which is incorporated herein by reference. In addition, the pharmaceutical compositions comprising peptides and proteins are described in, *e.g., Therapeutic Peptides and Proteins Formulations, Processing, and Delivery Systems,* by Ajay K. Banga, Technomic Publishing Company, Inc., Lancaster, PA (1995).

In a preferred embodiment, the pharmaceutical composition of the present invention is formulated for oral administration. In this embodiment, it is preferred that ADNF polypeptides comprising all D-amino acids are used. A pharmaceutically acceptable nontoxic composition is formed by incorporating any of normally employed excipients, and generally 10-95% of active ingredient and more preferably at a concentration of 25%-75%. Furthermore, to improve oral absorption of ADNF polypeptides, various carrier systems, such as nanoparticles, microparticles, liposomes, phospholipids, emulsions, erythrocytes, *etc.* can be used. The oral agents comprising ADNF polypeptides of the invention can be in any suitable form for oral administration, such as liquid, tablets, capsules, or the like. The oral formulations can be further coated or treated to prevent or reduce dissolution in stomach. *See, e.g., Therapeutic Peptides and Proteins, Formulation, Processing, and Delivery Systems,* by A.K. Banga, Technomic Publishing Company, Inc., 1995.

Furthermore, the ADNF polypeptides comprising at least one D-amino acid within the active core sites are embodied in pharmaceutical compositions intended for parenteral, topical, oral, sublingual, gavage, or local administration. For example, the pharmaceutical compositions are administered parenterally, *e.g.*, intravenously, subcutaneously, intradermally, or intramuscularly, or intranasally. Thus, the invention provides compositions for parenteral administration that comprise a solution of a mixture of ADNF polypeptides, dissolved or suspended in an acceptable carrier, preferably an aqueous carrier. A variety of aqueous carriers may be used including, for example, water, buffered water, 0.4% saline, 0.3% glycine, hyaluronic acid and the like. These compositions may be sterilized by conventional, well known sterilization techniques or, they may be sterile filtered. The resulting aqueous solutions may be packaged for use as is or lyophilized, the lyophilized preparation being combined with a sterile solution prior to administration. The compositions may contain pharmaceutically acceptable auxiliary substances as required to approximate physiological conditions including pH adjusting and buffering agents, tonicity adjusting agents, wetting agents and the like, such as, for example, sodium acetate, sodium lactate, sodium chloride, potassium chloride, calcium chloride, sorbitan monolaurate, triethanolamine oleate, *etc.*

For aerosol administration, ADNF polypeptides comprising at least one D-amino acid within the active core sites are preferably supplied in finely divided form along with a surfactant and propellant. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, *e.g.*, lecithin for intranasal delivery.

For solid compositions, conventional nontoxic solid carriers may be used. Solid carriers include, for example, pharmaceutical grades of mannitol, lactose, starch, magnesium stearate, sodium saccharin, talcum, cellulose, glucose, sucrose, magnesium carbonate, and the like.

Small polypeptides including SALLRSIPA and NAPVSIPQ cross the blood brain barrier. For longer polypeptides that do not cross blood the brain barrier, methods of administering proteins to the brain are well known. For example, proteins, polypeptides, other compounds and cells can be delivered to the mammalian brain via intracerebroventricular (ICV) injection or via a cannula (*see, e.g.,* Motta & Martini, *Proc. Soc. Exp*. *Biol*. *Med.* 168:62-64 (1981); Peterson *et al., Biochem. Pharmacol.* 31:2807-2810 (1982); Rzepczynski *et al., Metab. Brain Dis.* 3:211-216 (1988); Leibowitz *et al., Brain Res. Bull.* 21:905-912 (1988); Sramka *et al*., *Stereotact. Funct. Neurosurg.* 58:79-83 (1992); Peng *et al*., *Brain Res*. 632:57-67 (1993); Chem *et al*., *Exp*. *Neurol.* 125:72-81 (1994); Nikkhah *et al., Neuroscience* 63:57-72 (1994); Anderson *et al*., *J*. *Comp. Neurol.* 357:296-317 (1995); and Brecknell & Fawcett, *Exp*. *Neurol.* 138:338-344 (1996)). In particular, cannulas can be used to administer neurotrophic factors to mammals (*see, e.g.,* Motta & Martini, *Proc. Soc. Exp. Biol. Med.* 168:62-64 (1981) (neurotensin); Peng *et al., Brain Res.* 632:57-67 (1993) (NGF); Anderson *et al., J*. *Comp*. *Neurol*. 357:296-317 (1995) (BDNF, NGF, neurotrophin-3).

Alternatively, longer ADNF polypeptides that do not cross blood brain barrier can be coupled with a material which assists the ADNF polypeptide to cross the blood brain barrier and to traverse the plasma membrane of a cell, or the membrane of an intra-cellular compartment such as the nucleus. Cellular membranes are composed of lipid-protein bilayers that are freely permeable to small, nonionic lipophilic compounds and are inherently impermeable to polar compounds, macromolecules, and therapeutic or diagnostic agents. However, proteins and other compounds such as liposomes have been described, which have the ability to translocate polypeptides such as ADNF polypeptides across a cell membrane.

For example, "membrane translocation polypeptides" have amphiphilic or hydrophobic amino acid subsequences that have the ability to act as membrane-translocating carriers. In one embodiment, homeodomain proteins have the ability to translocate across cell membranes. The shortest intemalizable peptide of a homeodomain protein, Antennapedia, was found to be the third helix of the protein, from amino acid position 43 to 58 (*see, e.g.,* Prochiantz, *Current Opinion in Neurobiology* 6:629-634 (1996)). Another subsequence, the hydrophobic domain of signal peptides, was found to have similar cell membrane translocation characteristics (*see, e.g.,* Lin *et al., J*. *Biol. Chem*. 270:1 4255-14258 (1995)).

Examples of peptide sequences which can be linked to a ADNF polypeptide of the invention, for facilitating uptake of ADNF polypeptides into cells, include, but are not limited to: an 11 animo acid peptide of the tat protein of HIV (*see* Schwarze *et al., Science* 285:1569-1572 (1999)); a 20 residue peptide sequence which corresponds to amino acids 84-103 of the p16 protein (*see* Fahraeus *et al*., *Current* *Biology* 6:84 (1996)); the third helix of the 60-amino acid long homeodomain of Antennapedia (Derossi *et al*., *J*. *Biol*. *Chem.* 269:10444 (1994)); the h region of a signal peptide such as the Kaposi fibroblast growth factor (K-FGF) h region (Lin *et al*., *supra*); or the VP22 translocation domain from HSV (Elliot & O'Hare, *Cell* 88:223-233 (1997)). Other suitable chemical moieties that provide enhanced cellular uptake may also be chemically linked to ADNF polypeptides.

Toxin molecules also have the ability to transport polypeptides across cell membranes. Often, such molecules are composed of at least two parts (called "binary toxins"): a translocation or binding domain or polypeptide and a separate toxin domain or polypeptide. Typically, the translocation domain or polypeptide binds to a cellular receptor, and then the toxin is transported into the cell. Several bacterial toxins, including *Clostridium perfringens* iota toxin, diphtheria toxin (DT), *Pseudomonas* exotoxin A (PE), pertussis toxin (PT), *Bacillus anthracis* toxin, and pertussis adenylate cyclase (CYA), have been used in attempts to deliver peptides to the cell cytosol as internal or amino-terminal fusions (Arora *et al*., *J*. *Biol*. *Chem*., 268:3334-3341 (1993); Perelle *et al., Infect. Immun*., 61:5147-5156 (1993); Stenmark *et al*., *J*. *Cell Biol.* 113:1025-1032 (1991); Donnelly *et al*., *PNAS* 90:3530-3534 (1993); Carbonetti *et al., Abstr. Annu. Meet. Am. Soc. Microbiol.* 95:295 (1995); Sebo *et al., Infect. Immun.* 63:3851-3857 (1995); Klimpel *et al*., *PNAS U*.*S*.*A*. 89:10277-10281 (1992); and Novak *et al*., *J*. *Biol*. *Chem.* 267:17186-17193 1992)).

Such subsequences can be used to translocate ADNF polypeptides across a cell membrane. ADNF polypeptides can be conveniently fused to or derivatized with such sequences. Typically, the translocation sequence is provided as part of a fusion protein. Optionally, a linker can be used to link the ADNF polypeptides and the translocation sequence. Any suitable linker can be used, *e.g.*, a peptide linker.

The ADNF polypeptides can also be introduced into an animal cell, preferably a mammalian cell, via a liposomes and liposome derivatives such as immunoliposomes. The term "liposome" refers to vesicles comprised of one or more concentrically ordered lipid bilayers, which encapsulate an aqueous phase. The aqueous phase typically contains the compound to be delivered to the cell, i.e., an ADNF polypeptide.

The liposome fuses with the plasma membrane, thereby releasing the ADNF polypeptides into the cytosol. Alternatively, the liposome is phagocytosed or taken up by the cell in a transport vesicle. Once in the endosome or phagosome, the liposome either degrades or fuses with the membrane of the transport vesicle and releases its contents.

In current methods of drug delivery via liposomes, the liposome ultimately becomes permeable and releases the encapsulated compound (in this case, an ADNF polypeptide) at the target tissue or cell. For systemic or tissue specific delivery, this can be accomplished, for example, in a passive manner wherein the liposome bilayer degrades over time through the action of various agents in the body. Alternatively, active drug release involves using an agent to induce a permeability change in the liposome vesicle. Liposome membranes can be constructed so that they become destabilized when the environment becomes acidic near the liposome membrane (*see, e.g., PNAS* 84:7851 (1987); *Biochemistry* 28:908 (1989)). When liposomes are endocytosed by a target cell, for example, they become destabilized and release their contents. This destabilization is termed fusogenesis. Dioleoylphosphatidylethanolamine (DOPE) is the basis of many "fusogenic" systems.

Such liposomes typically comprise an ADNF polypeptide and a lipid component, *e.g.*, a neutral and/or cationic lipid, optionally including a receptor-recognition molecule such as an antibody that binds to a predetermined cell surface receptor or ligand (*e*.*g*., an antigen). A variety of methods are available for preparing liposomes as described in, *e.g.,* Szoka *et al*., *Ann*. *Rev. Biophys. Bioeng.* 9:467 (1980), U.S. Pat. Nos. 4,186,183, 4,217,344, 4,235,871, 4,261,975, 4,485,054, 4,501,728, 4,774,085, 4,837,028, 4,235,871, 4,261,975, 4,485,054, 4,541,728, 4,774,085, 4,837,028, 4,946,787, PCT Publication No. WO 91/17424, Deamer & Bangham, *Biochim*. *Biophys. Acta* 443:629-634 (1976); Fraley, *et al*., *PNAS* 76:3348-3352 (1979); Hope *et al*., *Biochim. Biophys. Acta* 812:55-65 (1985); Mayer *et al., Biochim. Biophys. Acta* 858:161-168 (1986); Williams *et al*., *PNAS* 85:242-246 (1988); *Liposomes* (Ostro (ed.), 1983, Chapter 1); Hope *et al*., *Chem*. *Phys. Lip.* 40:89 (1986); Gregoriadis, *Liposome Technology* (1984) and Lasic, *Liposomes: from Physics to Applications* (1993)). Suitable methods include, for example, sonication, extrusion, high pressure/homogenization, microfluidization, detergent dialysis, calcium-induced fusion of small liposome vesicles and ether-fusion methods, all of which are well known in the art.

In certain embodiments of the present invention, it is desirable to target the liposomes of the invention using targeting moieties that are specific to a particular cell type, tissue, and the like. Targeting of liposomes using a variety of targeting moieties (*e.g.*, ligands, receptors, and monoclonal antibodies) has been previously described (*see,* *e.g.*, U.S. Patent Nos. 4,957,773 and 4,603,044). Standard methods for coupling targeting agents to liposomes can be used. These methods generally involve incorporation into liposomes lipid components, *e.g.*, phosphatidylethanolamine, which can be activated for attachment of targeting agents, or derivatized lipophilic compounds, such as lipid derivatized bleomycin. Antibody targeted liposomes can be constructed using, for instance, liposomes which incorporate protein A (*see* Renneisen *et al*., *J*. *Biol*. *Chem*., 265:16337-16342 (1990) and Leonetti *et al*., *PNAS* 87:2448-2451 (1990).

Alternatively, nucleic acids encoding ADNF can also be used to provide a therapeutic dose of ADNF polypeptides. These nucleic acids can be inserted into any of a number of well-known vectors for the transfection of target cells and organisms. For example, nucleic acids are delivered as DNA plasmids, naked nucleic acid, and nucleic acid complexed with a delivery vehicle such as a liposome. Viral vector delivery systems include DNA and RNA viruses, which have either episomal or integrated genomes after delivery to the cell. For a review of gene therapy procedures, *see* Anderson, *Science* 256:808-813 (1992); Nabel & Felgner, *TIBTECH* 11:211-217 (1993); Mitani & Caskey, *TIBTECH* 11:162-166 (1993); Dillon, *TIBTECH* 11:167-175 (1993); Miller, *Nature* 357:455-460 (1992); Van Brunt, *Biotechnology* 6(10):1149-1154 (1988); Vigne, *Restorative Neurology and Neuroscience* 8:35-36 (1995); Kremer & Perricaudet, *British Medical Bulletin* 51(1):31-44 (1995); Haddada *et al.,* in *Current Topics in Microbiology and Immunology* Doerfler and Böhm (eds) (1995); and Yu *et al*., *Gene Therapy* 1:13-26 (1994).

Methods of non-viral delivery of nucleic acids include lipofection, microinjection, biolistics, virosomes, liposomes, immunoliposomes, polycation or lipid:nucleic acid conjugates, naked DNA, artificial virions, *and* agent-enhanced uptake of DNA. Lipofection is described in, *e.g.,* U.S. Patent No. 5,049,386, U.S. Patent No. 4,946,787; and U.S. Patent No. 4,897,355) and lipofection reagents are sold commercially (*e*.*g*., Transfectam™ and Lipofectin™). Cationic and neutral lipids that are suitable for efficient receptor-recognition lipofection of polynucleotides include those of Felgner, WO 91/17424, WO 91/16024. Delivery can be to cells (*ex vivo* administration) or target tissues (*in vivo* administration).

In therapeutic applications, ADNF polypeptides of the invention are administered to a patient in an amount sufficient to reduce neuronal cell death associated with various disorders, to reduce oxidative stress in a patient, or to reduce a condition associated with fetal alcohol syndrome in a subject *in utero.* An amount adequate to accomplish this is defined as a "therapeutically effective dose." Amounts effective for this use will depend on, for example, the particular ADNF polypeptide employed, the conditions to be treated, the type of neuronal cell death or damage to be prevented, the manner of administration, the weight and general state of health of the patient, and the judgment of the prescribing physician. For example, for the prevention or reduction of neuronal cell death, an amount of ADNF polypeptides falling within the range of a 1µg to 50 µg, preferably 1µg to 10µg dose given orally once a day per mouse (*e*.*g*., in the evening) would be a therapeutically effective amount. This dose is based on the average body weight of mice, and an appropriate dose for human can be extrapolated based on the average weight of human.

### IV. USE OF THE ADNF POLYPEPTIDES OF THE PRESENT INVENTION FOR THE MANUFACTURE OF A MEDICAMENT FOR REDUCING NEURONAL CELL DEATH

In another aspect, the present invention provides use of the ADNF polypeptides of the present invention for the manufacture of a medicament for reducing neuronal cell death, comprising contacting neuronal cells with an ADNF polypeptide in an amount sufficient to reduce neuronal cell death, wherein the ADNF polypeptide comprises at least one D-amino acid within its active core site, preferably at the N-terminus and/or the C-terminus of the active core site. In this use, the ADNF polypeptide can be an ADNF I polypeptide, an ADNF III polypeptide, or mixtures thereof.

In one embodiment, the use comprises contacting neuronal cells with an ADNF polypeptide, wherein the ADNF polypeptide is a member selected from the group consisting of: (a) an ADNF I polypeptide comprising an active core site having the following amino acid: Ser-Ala-Leu-Leu-Arg-Ser-Ile-Pro-Ala (SEQ ID NO:1); (b) an ADNF III polypeptide comprising an active core site having the following amino acid: Asn-Ala-Pro-Val-Ser-Ile-Pro-Gln (SEQ ID NO:2); and (c) a mixture of the ADNF I polypeptide or part (a) and the ADNF III polypeptide of part (b); wherein at least one of the ADNF I polypeptide and the ADNF III polypeptide comprises an active core site comprising at least one D-amino acid.

In another embodiment, the use comprises contacting neuronal cells with an ADNF I polypeptide, wherein the active core site of the ADNF I polypeptide comprises at least one D-amino acid, preferably at the N-terminus and/or the C-terminus of the active core site. The previous discussion pertaining to the location and the number of D-amino acids within the active core site of ADNF I, as well as the discussion of additional D- and/or L-amino acids added on to the active site of the ADNF I polypeptide is fully applicable, and thus, will not be repeated with respect to this particular embodiment of the invention.

In yet another embodiment, the use comprises contacting neuronal cells with an ADNF III polypeptide, wherein the active core site of the ADNF III polypeptide comprises at least one D-amino acid, preferably at the N-terminus and/or the C-terminus of the active core site. The previous discussion pertaining to the location and the number of D-amino acids within the active core site of ADNF III, as well as the discussion of additional D- and/or L-amino acids added on to the active site of the ADNF III polypeptide is fully applicable, and thus, will not be repeated with respect to this particular embodiment of the invention.

In yet another embodiment, the use comprises contacting neuronal cells with a mixture of an ADNF I polypeptide and an ADNF III polypeptide, wherein at least one of the ADNF I polypeptide and the ADNF III polypeptide comprises an active core site comprising at least one D-amino acid. The previous discussion pertaining to the location and the number of D-amino acids within the active core site of ADNF I or ADNF III, as well as the discussion of additional D- and/or L-amino acids added on to the active site of the ADNF I polypeptide or ADNF III polypeptide is fully applicable, and thus, will not be repeated with respect to this particular embodiment of the invention.

ADNF polypeptides of the present invention can be used for manufacture of a medicament for the treatment of neurological disorders and for the prevention of neuronal cell death. For example, ADNF polypeptides of the present invention can be used to prevent the death of neuronal cells including, but not limited to, spinal cord neurons, hippocampal neurons, cerebral cortical neurons and cholinergic neurons. More particularly, ADNF polypeptides of the present invention can be used in the prevention of cell death associated with (1) gp120, the envelope protein from HIV; (2) *N*-methyl-D-aspartic acid (excito-toxicity); (3) tetrodotoxin (blockage of electrical activity); and (4) β-amyloid peptide, a substance related to neuronal degeneration in Alzheimer's disease.

As such, the ADNF polypeptides of the present invention can be used to reduce gp120-induced neuronal cell death by administering an effective amount of an ADNF polypeptide of the present invention to a patient infected with the HIV virus. The ADNF polypeptides of the present invention can also be used to reduce neuronal cell death associated with excito-toxicity induced by N-methyl-D-aspartate stimulation, the use comprising contacting neuronal cells with an ADNF polypeptide of the present invention in an amount sufficient to prevent neuronal cell death. The ADNF polypeptides of the present invention can also be used to reduce cell death induced by the β-amyloid peptide in a patient afflicted or impaired with Alzheimer's disease, the use comprising administering to the patient an ADNF polypeptide of the present invention in an amount sufficient to prevent neuronal cell death. The ADNF polypeptides can also be used to alleviate learning impairment produced by cholinergic blockage in a patient afflicted or impaired with Alzheimer's disease. For example, ADNF polypeptides can be used to improve short-term and/or reference memory in Alzheimer's patients.

Similarly, it will be readily apparent to those of skill in the art that the ADNF polypeptides of the present invention can be used in a similar manner to prevent neuronal cell death associated with a number of other neurological diseases and deficiencies. Pathologies that would benefit from therapeutic and diagnostic applications of this invention include conditions (diseases and insults) leading to neuronal cell death and/or sub-lethal neuronal pathology including, for example, the following:
diseases of central motor systems including degenerative conditions affecting the basal ganglia (Huntington's disease, Wilson's disease, striatonigral degeneration, corticobasal ganglionic degeneration), Tourette's syndrome, Parkinson's disease, progressive supranuclear palsy, progressive bulbar palsy, familial spastic paraplegia, spinomuscular atrophy, ALS and variants thereof, dentatorubral atrophy, olivo-pontocerebellar atrophy, paraneoplastic cerebellar degeneration, and dopamine toxicity;
diseases affecting sensory neurons such as Friedreich's ataxia, diabetes, peripheral neuropathy, retinal neuronal degeneration;
diseases of limbic and cortical systems such as cerebral amyloidosis, Pick's atrophy, Retts syndrome;
neurodegenerative pathologies involving multiple neuronal systems and/or brainstem including Alzheimer's disease, AIDS-related dementia, Leigh's disease, diffuse Lewy body disease, epilepsy, multiple system atrophy, Guillain-Barre syndrome, lysosomal storage disorders such as lipofuscinosis, late-degenerative stages of Down's syndrome, Alper's disease, vertigo as result of CNS degeneration;
pathologies associated with developmental retardation and learning impairments, and Down's syndrome, and oxidative stress induced neuronal death;
pathologies arising with aging and chronic alcohol or drug abuse including, for example, with alcoholism the degeneration of neurons in locus coeruleus, cerebellum, cholinergic basal forebrain; with aging degeneration of cerebellar neurons and cortical neurons leading to cognitive and motor impairments; and with chronic amphetamine abuse degeneration of basal ganglia neurons leading to motor impairments;
pathological changes resulting from focal trauma such as stroke, focal ischemia, vascular insufficiency, hypoxic-ischemic encephalopathy, hyperglycemia, hypoglycemia, closed head trauma, or direct trauma;
pathologies arising as a negative side-effect of therapeutic drugs and treatments (*e.g.*, degeneration of cingulate and entorhinal cortex neurons in response to anticonvulsant doses of antagonists of the NMDA class of glutamate receptor).

### V. USE OF ADNF PEPTIDES OF THE PRESENT INVENTION FOR THE MANUFACTURE OF A MEDICAMENT FOR REDUCING OXIDATIVE STRESS

In yet another aspect, the present invention provides use of ADNF peptides of the present invention for the manufacture of a medicament for treating oxidative stress in a patient by administering to the patient an ADNF polypeptide in an amount sufficient to prevent or reduce oxidative stress, wherein the ADNF polypeptide comprises at least one D-amino acid within its active core site, preferably at the N-terminus and/or the C-terminus of the active core site. Oxidative stress has been implicated in several neurodegenerative diseases in humans (Cassarmno & Bennett, *Brain Res. Reviews* 29:1-25 (1999)). Moreover, oxidative stress produced from alcohol administration has been associated with fetal death and abnormalities (*e.g.*, conditions associated with fetal alcohol syndrome). *See, e.g.,* Henderson *et al., Alcoholism: Clinical and Experimental Research* 19:714-720 (1995). In these uses the ADNF polypeptide can be an ADNF I polypeptide, an ADNF III polypeptide, or mixtures thereof. By using the ADNF polypeptides of the present invention, oxidative stress associated with various clinical conditions can be reduced.

In one embodiment, the use comprises treating oxidative stress in a patient with an ADNF polypeptide, wherein the ADNF polypeptide is a member selected from the group consisting of: (a) an ADNF I polypeptide comprising an active core site having the following amino acid: Ser-Ala-Leu-Leu-Arg-Ser-Ile-Pro-Ala (SEQ ID NO:1); (b) an ADNF III polypeptide comprising an active core site having the following amino acid: Asn-Ala-Pro-Val-Ser-Ile-Pro-Gln (SEQ ID NO:2); and (c) a mixture of the ADNF I polypeptide or part (a) and the ADNF III polypeptide of part (b); wherein at least one of the ADNF I polypeptide and the ADNF III polypeptide comprises an active core site comprising at least one D-amino acid, preferably at the N-terminus and/or the C-terminus of the active core site.

In another embodiment, the use comprises treating oxidative stress in a patient with an ADNF I polypeptide, wherein the active core site of the ADNF I polypeptide comprises at least one D-amino acid, preferably at the N-terminus and/or the C-terminus of the active core site. The previous discussion pertaining to the location and the number of D-amino acids within the active core site of ADNF I, as well as the discussion of additional D- and/or L-amino acids added on to the active site of the ADNF I polypeptide is fully applicable, and thus, will not be repeated with respect to this particular embodiment of the invention.

In yet another embodiment, the use comprises treating oxidative stress in a patient with an ADNF III polypeptide, wherein the active core site of the ADNF III polypeptide comprises at least one D-amino acid, preferably at the N-terminus and/or the C-terminus of the active core site. The previous discussion pertaining to the location and the number of D-amino acids within the active core site of ADNF III, as well as the discussion of additional D- and/or L-amino acids added on to the active site of the ADNF III polypeptide is fully applicable, and thus, will not be repeated with respect to this particular embodiment of the invention.

In yet another embodiment, the method comprises treating oxidative stress in a patient with a mixture of an ADNF I polypeptide and an ADNF III polypeptide, wherein at least one of the ADNF I and the ADNF III polypeptide comprises an active core site comprising at least one D-amino acid. The previous discussion pertaining to the location and the number of D-amino acids within the active core site of ADNF I or ADNF III, as well as the discussion of additional D- and/or L-amino acids added on to the active site of the ADNF I or ADNF III polypeptide is fully applicable, and thus, will not be repeated with respect to this particular embodiment of the invention.

### VI. USE OF THE ADNF POLYPEPTIDES OF THE PRESENT INVENTION FOR THE MANUFACTURE OF A MEDICAMENT FOR REDUCING A CONDITION ASSOCIATED WITH FETAL ALCOHOL SYNDROME

In yet another aspect, the present invention provides a use of the ADNF polypeptides of the present invention for the manufacture of a medicament for reducing a condition associated with fetal alcohol syndrome in a subject who is exposed to alcohol *in utero*, the use comprising administering to the subject an ADNF polypeptide in an amount sufficient to reduce the condition associated with fetal alcohol syndrome, wherein the ADNF polypeptide comprises an active core site comprising at least one D-amino acid, preferably at the N-terminus and/or the C-terminus of the active core site. In this use, the ADNF polypeptide can be an ADNF I polypeptide, an ADNF III polypeptide, or mixtures thereof.

Treatment of a well-characterized model for FAS (*e.g.*, C57B1/6J mouse strain) with an ADNF polypeptide comprising at least one D-amino acid within an active core site reduces or prevents alcohol induced fetus death, body and brain weight reduction, and VIP mRNA reduction. Similarly, the human embryo, fetus, or subject can be protected from alcohol induced effects by administering an ADNF polypeptide directly to the embryo, fetus, or subject, or by administering the ADNF polypeptide indirectly to the fetus by administering it to the mother. Preferably, ADNF polypeptides are orally administered.

In one embodiment, the use comprises administering to a subject who is exposed to alcohol *in utero* with an ADNF polypeptide, wherein the ADNF polypeptide is a member selected from the group consisting of: (a) an ADNF I polypeptide comprising an active core site having the following amino acid: Ser-Ala-Leu-Leu-Arg-Ser-Ile-Pro-Ala (SEQ ID NO:1); (b) an ADNF III polypeptide comprising an active core site having the following amino acid: Asn-Ala-Pro-Val-Ser-Ile-Pro-Gln (SEQ ID NO:2); and (c) a mixture of the ADNF I polypeptide or part (a) and the ADNF III polypeptide of part (b); wherein at least one of the ADNF I polypeptide and the ADNF III polypeptide comprises an active core site comprising at least one D-amino acid.

In another embodiment, the use comprises administering to a subject who is exposed to alcohol *in utero* with an ADNF I polypeptide, wherein the active core site of the ADNF I polypeptide comprises at least one D-amino acid, preferably at the N-terminus and/or the C-terminus of the active core site. The previous discussion pertaining to the location and the number of D-amino acids within the active core site of ADNF I, as well as the discussion of additional D- and/or L-amino acids added on to the active site of the ADNF I polypeptide is fully applicable, and thus, will not be repeated with respect to this particular embodiment of the invention.

In yet another embodiment, the use comprises administering to a subject who is exposed to alcohol *in utero* with an ADNF III polypeptide, wherein the active core site of the ADNF III polypeptide comprises at least one D-amino acid, preferably at the N-terminus and/or the C-terminus of the active core site. The previous discussion pertaining to the location and the number of D-amino acids within the active core site of ADNF III, as well as the discussion of additional D- and/or L-amino acids added on to the active site of the ADNF III polypeptide is fully applicable, and thus, will not be repeated with respect to this particular embodiment of the invention.

In yet another embodiment, the use comprises administering to a subject who is exposed to alcohol *in utero* with a mixture of an ADNF I polypeptide and an ADNF III polypeptide, wherein at least one of the ADNF I and the ADNF III polypeptide comprises an active core site comprising at least one D-amino acid. The previous discussion pertaining to the location and the number of D-amino acids within the active core site of ADNF I or ADNF III, as well as the discussion of additional D- and/or L-amino acids added on to the active site of the ADNF I or the ADNF III polypeptide is fully applicable, and thus, will not be repeated with respect to this particular embodiment of the invention.

### EXAMPLES

### A. In vitro experiments

Dissociated cerebral cortical cultures prepared as described (Brenneman & Gozes, *J*. *Clin. Invest.* 97:2299-2307 (1996)) were used to compare the survival-promoting actions of ADNF I and ADNF III derived peptides. Comparisons were made with the D-form of the peptide and in combination with the L-form of the peptides. The test paradigm consisted of the addition of the test peptide in cultures that were co-treated with tetrodotoxin (TTX). TTX produced an apoptotic death in these cultures and is used as a model substance to demonstrate efficacy against this "programmed cell death" and all other means that produce this type of death mechanism. The duration of the test period was 5 days, and neurons were counted and identified by characteristic morphology and by confirmation with an immunocytochemical marker for neurons: neuron specific enolase.

As shown in Figure 1, the D- and L-forms of SALLRSIPA (SAL) were identical in both potency and efficacy in preventing neuronal cell death associated with electrical blockade with TTX. Each point is the mean of at least three determinations, the error bars are the standard errors. Similarly, the D- and L-forms of NAPVSIPQ (NAP) were very similar, with each exhibiting a complex dose response with two apparent maxima (Figure 2A). Unless indicated as otherwise, L-SAL and D-SAL refer to a peptide having an amino acid sequence of Ser-Ala-Leu-Leu-Arg-Ser-Ile-Pro-Ala (SEQ ID NO:1) comprising all L-amino acids or all D-amino acids, respectively. Also, L-NAP and D-NAP refer to a peptide having an amino acid sequence of Asn-Ala-Pro-Val-Ser-Ile-Pro-Gln (SEQ ID NO:2) comprising all L-amino acids or all D-amino acids, respectively.

In Figure 2B, the effect of an ADNF peptide that has amino acid residues in both L-form and in D-form, namely D-NA{L-P}VSIPQ, was tested. In this ADNF peptide, all of the amino acids of NAPVSIPQ were in the D-form, except the third proline residue was in the L-form. Cerebral cortical cultures were treated with 1 µM TTX for 5 days, which is a model of apopotic death that is relevant to neurodegenerative disease. Cultures treated with the toxin were given various concentrations of D-NA{L-P}VSIPQ. As all L- and all D- amino acid NAPVSIPQ, this mixed D/L peptide D-NA {L-P} VSIPQ retained survival-promoting activity and was effective in cell culture in preventing neuronal cell death in the TTX model.

As illustrates in Figures 3A and 3B, combinations of peptides were also tested. For all combinatorial experiments, the two peptides are given in equimolar amounts. In Figure 3A, the effect of D-NAP and D-SAL was shown to produce a different dose response from that observed with either agent alone. Importantly, there was no apparent attenuation of the survival-promoting activity at higher concentration of peptide. This apparent synergy between the peptides is significant because it indicates that there may be a broader therapeutic range of effective concentrations if both D-peptides are used combinatorially. Similar experiments conducted with both L-SAL and L-NAP resulted in significant loss of efficacy, although synergy was still evident (Figure 3A).

Another series of experiments were conducted to show the effect of combining L- and D-forms of NAP and SAL. As shown in Figure 3B, the use of L-NAP and D-SAL showed full efficacy and high potency in preventing apoptotic death of neurons treated with TTX. There was no apparent attenuation of the protective activity at high concentrations (> 1 pM) of peptide; *i.e.*, synergy was again evident. In contrast, treatment with D-NAP and L-SAL resulted in full efficacy but attenuation of the survival-promoting activity at concentration > 0.1 pM. These data indicate specificity for combinations of D and L- peptides.

Figure 4 illustrates that ADNF polypeptides can protect against beta amyloid toxicity *in vitro.* PC12 cells (Solomon et al., *Proc*. *Natl*. *Acad. Sci. USA* 94: 4109-4112 (1997)) were maintained in DMEM (Dulbecco's modified Eagles medium) supplemented with 8% horse serum, 8% heat-inactivated fetal calf serum, 2mM L-Glutamine, (all purchased from Sigma, Rehovot, Israel), 100 mgr/ml streptomycin and 100 U/l penicillin (Biological Industries, Beit Haemek, Israel). Cultures were maintained at 37°C/5% CO₂ as monolayers in 75 cm² flasks and were split at a 1:12 ratio twice a week. For the beta amyloid (amino acids 25-35) treatment, seeding was at 1.5x10⁵ cells/ml on 96-well plates (100 ml/well) in a medium containing: DMEM supplemented with 1% Penicillin/ Streptomycin, 0.5M insulin (Sigma, Rehovot, Israel). Twenty-four hours after the addition of peptides 10-9 M (D-SAL and D-NAP in a 1:1 mixture diluted to final concentration of 1 nM), beta amyloid was added at 2.5 mM and cell viability (metabolic activity) was measured 48 hours later. Metabolic activity was measured by a colorimetric method using a tetrazolium compound (3-(4,5-dimethylthiazol-2-yl-5-(3-carboxymethoxyphenyl )-2- (4-sulfophenyl) -2H tetrazlium (MTS) and an electron coupling reagent phenazine methasulfate. MTS is bioreduced by the living cells to the Formazan form, that is detected at 490 nm (Promega, Medison WI, USA). Results showed significant activity loss in the presence of the toxin and protection by the peptides D-SAL+D-NAP.

### B. In vivo experiments

A variety of experimental models were utilized to demonstrate the efficacy of D-NAP and D-SAL in animals. Various routes of administration were employed in both rats and mice.

### 1. Fetal alcohol syndrome (FAS) in mice.

A well established model for FAS was used to test the efficacy of ADNF I and ADNF III peptides in mice (Webster *et al*., *Neurobehav. Tox.* 2:227-234 (1980), incorporated herein by reference). This test is designed to test for efficacy against severe oxidative stress produced from alcohol administration (Amini *et al*., *Free Radical Biology and Medicine* 21:357-365 (1996); Schlorff *et al., Alcohol* 17:97-105 (1999)). Fetal death and abnormalities are associated with the generation of free radicals and oxidative damage (Henderson *et al., Alcoholism: Clinical and Experimental Research* 19:714-720 (1995)). The model was chosen in that it allowed for a rapid and relevant evaluation of agents efficacious against severe oxidative stress. Since oxidative stress has been implicated in several neurodegenerative diseases in humans (Cassarmno & Bennett, *Brain Res. Reviews* 29:1-25 (1999)), efficacy in FAS can be of predictive value in the treatment of human disease.

A single injection of 25% ethyl alcohol in saline was given intraperitoneally at 0.030 ml/g body weight to pregnant mice at embryonic day 8. In the first series of experiments, peptides were given 30 minutes prior to the administration of alcohol. Dosages of 2 µg or 20 µg were given. D-NAP or L-NAP (0.5 mg) was dissolved in 50 µl of dimethyl sulfoxide and diluted with filtered (0.22µ) Dulbecco's phosphate buffered saline (DPBS) to a final volume of 5 ml. The injection volume was 200 µl. D-SAL or L-SAL was dissolved in DPBS before administration. The litter mean was used as a single measurement for statistical analysis. The average litter size was 8 and it did not differ among treatment groups.

Evaluation of the surviving fetuses was done on embryonic day 18. Evaluation of efficacy was by the number of surviving fetuses (Figure 5), brain weight (Figure 6A) and total fetal body weight (Figure 6B). As shown in Figure 5, treatment with alcohol resulted in 37% fetal demise in comparison to 6% in controls. Pretreatment with 20 µg D-NAP, D-SAL or L-NAP + D-SAL (20 µg each) significantly reduced the fetal demise rate in comparison to those in the alcohol group (P < 0.03). As shown in Figure 6A, of the surviving fetuses whose mother had been treated with alcohol, only those co-treated with L-NAP and D-SAL had significantly greater brain weights in comparison to those in the alcohol group. Similar protective effects of L-NAP and D-SAL were evident as assessed by total fetal weights (Figure 6B).

To test for a critical period of peptide administration that could still produce an effective intervention, L-NAP (20 µg) + L-SAL (20 µg) were administered one hour or three hours after alcohol treatment of pregnant mice at gestational day E8. As shown in Figure 7, post-treatment at 1 hour with NAP + SAL prevented the demises observed with alcohol treatment; however, post-treatment at 3 hours did not result in significant prevention of fetal demise to control levels. In addition, post-treatment with NAP + SAL (1 hour and 3 hours) prevented the microcephaly (Figure 8), but not the growth restriction associated with FAS.

To demonstrate that D-NAP and D-SAL were effective through oral administration, the peptides were given by gavage (*i.e.,* introducing peptides into the stomach by a tube) to pregnant mice at gestational age day 8. As shown in Figure 9, a significant increase in fetal survival was observed after oral treatment with 40 µg each of D-NAP and D-SAL. This is the first demonstration of an orally active embryo-protecting action of a peptide.

Figures 10A and 10B illustrates effects of oral administration of ADNF polypeptides on pup brain weight and fetal death. Pregnant mice were injected with alcohol as a model for fetal alcohol syndrome according to methods of Webster et al. (1980), *supra.* The pregnant mice were injected 25% alcohol at 0.030 ml/g body weight. Peptide was dissolved in phosphate-buffered saline and administered orally by gavage 30 minutes prior to alcohol treatment. D-SAL (all D-amino acids of SALLRSIPA) at 40 µg was found to prevent fetal death as assessed on E18.

### 2. Apo E knockout mice: developmental behavior assays

Recent studies have demonstrated that the inheritance of the lipid carrier apolipoprotein E4 (ApoE4) is a major risk factor in Alzheimer's disease (Strittmatter & Roses, *Proc. Natl*. *Acad Sci. USA* 92:4725-4727 (1995)). These studies, along with the investigations of ApoE-deficient animals indicated that an apolipoprotein E functioning system is required for normal neurodevelopment and function (Masliah *et al*., *J*. *Exp. Neurol.* 136:107-122 (1995)). The acquisition of developmental milestones of behavior requires appropriate synapse formation and proper brain conductivity (Altman *et al., Anim. Behav.* 23:896-920 (1975)). ApoE-deficient animals have been shown to be developmentally retarded (Gozes *et al*., *J*. *Neurobiol.* 33: 329-342 (1997), incorporated herein by reference) offer a test system for the *in vivo* effects of putative neurotrophic substances, such as D-SAL and D-NAP.

Newborn animals were tested for the onset neurobehavioral developmental milestones as previously described (Gozes *et al., J*. *Neurobiol.* 33: 329-342 (1997); Bassan *et al., J*. *Neurochem.* 72: 1283-1293 (1999)). For these experiments, animals were treated either by oral application, or subcutaneous injection of D-SAL + D-NAP. Peptides (0.5 mg each) were dissolved in 0.01M acetic acid (30 microliters). For both applications, 0.5 microgram of each of the test drugs were delivered; for the oral application (sublingual), in 10 microliter saline and for the injection in 20 microliters. This protocol was used for the first 4 days of life. From day 5-10, the amount of the peptides and the solution volume was doubled. From day 11-14, the amount of peptide was 2 microgram each in 40 microliter (oral) and 80 microliter (injection). Tests performed daily included cliff avoidance, negative geotaxis, placing and righting behaviors. Both subcutaneous and oral administration of D-NAP and D-SAL were compared. As shown in Figure 11, the slowest responders for cliff avoidance were the apoE knockout animals. This confirms previous studies which show that the behavioral developmental and learning is delayed in these animals in comparison to control animals (Gozes *et al*., *J*. *Neurobiol*. 33:329-342 (1997); Bassan *et al., J*. *Neurochem.* 72:1283-1293 (1999)). Administration of D-NAP + D-SAL by either subcutaneous injection or oral administration resulted in significant increases in the behavioral score, indicative of a more rapid acquisition of this developmental milestone. Similar effects were observed for negative geotaxis (Figure 12) and placing behavior (Figure 13).

More detailed evaluation of the results is as follows. In the following, one way analysis of variance with multiple comparison of the means (Student-Newman-Keuls method) were used for statistical comparisons.
1. Figure 11 (cliff avoidance): the difference between apoE-deficient mice and control animals was apparent only on the fifth day of life (P<0.001). Injection ofD-peptides to control, resulted in no effect, while injection to the deficient mice resulted in an effect only on the third day. Oral application resulted in a significant improvements only on the first day, in the deficient mice.
2. Figure 12 (negative geotaxis): While there was no difference on the first day between control and apoE-deficient (with perhaps a difference on day three, P<0.006), treatment of the latter (injection or oral) resulted in significant improvements with injection on days 1, 2, 4 and 5, and with oral treatment on days 1 and 5. (P<0.001).
3. Figure 13 (placing): The difference between apoE-deficient mice and control animals was apparent only on the first day of life (P<0.001). Similarly, oral application of the peptide mixture was efficient in enhancing the response only on the first day of life.

### 3. AF64A cholinotoxicity in adult rats

Another focus of the present invention are the neuroprotective properties of the D-SAL and D-NAP in animals exposed to the cholinotoxin, ethylcholine aziridium (AF64A), a blocker of choline uptake (Fisher *et al*., *Neurosci. Lett*. 102:325-331 (1989)). An intact cholinergic system is required for normal brain function, whereas Alzheimer's disease is associated with the death of cholinergic cells (Brumback & Leech, *J*. *Okla. State Med. Assoc.* 87:103-111 (1994)). Rats treated with AF64A provide an accepted model for testing *in vivo* efficacy of cholinergic-enhancing drugs.

Although the identity of the ADNF-dependent neurons has not been fully characterized, previous studies indicated that some cholinergic neurons are among those affected (Gozes *et al*., *Brain Res. Dev.* 99:167-175 (1997)). In this context, ApoE-deficient mice (described above) exhibited reduced choline acetyl transferase activity (Gordon *et al*., *Neurosci. Lett*. 199:1-4 (1995); Gozes *et al., J*. *Neurobiol.* 33:329-342 (1997)) and treatment with L-NAP significantly increased cholinergic function to control levels (Bassan *et al*., *J*. *Neurochem.* 72:1283-1293 (1999)) while L-SAL treatment was less effective.

Rats (male Wistar, 300-350g) were subjected to two daily tests in a water maze, including a hidden platform (Morris, *J*. *Neurosci. Methods* 11:47-60 (1984) and Gordon *et al*., *Neurosci. Lett*. 199:1-4 (1995); Gozes *et al., J*. *Neurobiol.* 33:329-342 (1997)). Every day for the first test, both the platform and the animal were situated in a new location with regard to the pool (with the pool being immobile). The experiment was performed as follows: the animal was positioned on the platform for 0.5 minutes then placed in the water. The time required to reach the platform (indicative of learning and intact reference memory) was measured (first test). After 0.5 minute on the platform, the animal was placed back in the water (in the previous position) for an additional second test and search for the hidden platform (retained in the previous position). The time required to reach the platform in the second trial was recorded, indicative of short-term (working) memory. All measurements were performed using the computerized video-assisted HVS water maze system (HVS Image Ltd. Hampton, UK). Animals were tested for four days to eliminate random memory defective animals. The best performers were injected i.c.v. at a rate of 0.21 µl/min. with the cholinotoxin ethylcholine aziridium (AF64A, 3 nmolI2 µl/side), control animals received an injection of saline (Gozes *et al*., *Proc. Natl*. *Acad*. *Sci. USA* 93:427-432 (1996)).

Animals were allowed to recover for one week, followed by daily exposure to three micrograms of D-SAL + three micrograms of D-NAP, in 20 microliter saline, applied orally on the tongue. After a week of oral peptide application, the animals were subjected to two daily tests in the water maze (as above). During the test-period, animals were also given an oral administration of peptide or vehicle (carrier) an hour before the daily test. It was previously shown that AF64A-treated animals exhibit learning and memory deficits in the Morris water maze test (Gozes *et al*., *Proc. Natl. Acad Sci. USA* 93:427-432 (1996)). Here, AF64 A-treated rats subjected to oral application of D-NAP + D-SAL exhibited a decreased latency in finding the hidden platform, indicative of improved reference memory (Figure 14A, first daily test). Furthermore, the same rats exhibited improved working memory in the second daily test (Figure 14B). These data indicate that oral administration of a combination of D-SAL and D-NAP resulted in significant increases in learning and memory in animals with chemically induced cholinergic impairment.

Figure 14C illustrates the effect of oral administration of D-SALLRSIPA alone on learning and memory in rats treated with the cholinotoxin AF-64A. Rats were treated with the cholinotoxin AF-64A and D-SALLRSIPA as described in Gozes et al., *J*. *Pharmacol. Exp. Therap.* 293: 1091-1098 (2000), except that D-SALLRSIPA was delivered to AF64A-treated rats was as follows: 10 microgram D-SALLRSIPA (D-SAL) per rat (250-300 g) per day in 50 microliter saline under the tongue, using a micropipette. Peptides were applied once daily for three days, a week after the AF64A lesion. After a 2-day cessation, peptides were applied once daily for another 5 days and tested from day three on. Following an additional two-day cessation, peptides were applied again daily for two days and tested in the Morris water maze. The graph shows the results of the 5 day testing. In each day the animals were subjected to two consecutive tests and results are a summation of the two daily tests. Significance (one way ANOVA with Student-Neuman-Kuels multiple comparison of means test) is as follows.
Day 1: P < 0.04 D-SALLRSIPA-AF64A vs. AF64A;
Day 2: P< 0.04 AF64A vs. control (sham operated), SALLRSIPA treatment was not significantly different from either AF64A animals or control, suggesting some improvement;
Day 3: No difference;
Day 4: No difference; and
Day 5: t-test: P < 0.04 D-SALLRSIPA-AF64A vs. AF64A.
These results suggest that D-SALLRSIPA (D-SAL) is effective on its own.

### 4. Memory improvements in ApoE-deficient mice:

Memory deficits and cholinergic impairments have been described in adult ApoE-deficient mice. These deficits may mimic the conditions found in people that are homozygous for apolipoprotein E4, a condition that in which patients are more prone to an early onset of Alzheimer's disease, in contrast to people carrying the E2 or E3 alleles (Gordon *et al*., *Neurosci*. *Lett*. 199:1-4 (1995)). A week after cessation of treatment, cognitive functions were assessed in the Morris water maze. Improvements of cognitive functions were observed a week after cessation of the 2-week daily D-SAL-D-NAP treatment, *i.e.* in 21-day-old mice exposed to a 5-day training protocol (Figure 15). Short-term memory processes were examined by performance in the water maze, measuring the time required to find the hidden platform in the second of two daily trials. The platform location and the starting point in which the animal was placed in the water were held constant within each pair of daily trials, but both locations were changed every day. On the second test of the first trial day, the ApoE-deficient mice were significantly retarded as compared to controls (P<0.04) and improved after oral application of D-SAL + D-NAP, with most of the treated animals finding the platform at a latency of ≤20 sec.

Figures 16A and 16B illustrate the first test and second test, respectively, of Morris water maze test results in apolipoprotein E-deficient mice. Experiments were performed following injections of a mixture of D-NAP-D-SAL with an injection protocol and Morris water maze as described in Gozes et al., *J*. *Pharmacol. Exp. Therap.* 293: 1091-1098 (2000)). Results showed significant improvements on day 1 and day 2 (first daily test, and on day three, second daily test)- P<0.05.

## Claims

1. An Activity Dependent Neurotrophic Factor I (ADNF I) polypeptide, the ADNF I polypeptide comprising an active core site having the following amino acid sequence: Ser-Ala-Leu-Leu-Arg-Ser-Ile-Pro-Ala (SEQ ID NO:1), wherein the active core site comprises at least one D-amino acid.

2. The ADNF I polypeptide of claim 1, wherein either an N-terminal amino acid or a C-terminal amino acid of the active core site is a D-amino acid.

3. The ADNF I polypeptide of claim 1, wherein both N-terminal and C-terminal amino acids of the active core site are D-amino acids.

4. The ADNF I polypeptide of claim 1, wherein the active core site comprises all D-amino acids.

5. The ADNF I polypeptide of claim 1, wherein the ADNF I polypeptide is Ser-Ala-Leu-Leu-Arg-Ser-Ile-Pro-Ala (SEQ ID NO:1).

6. The ADNF I polypeptide of claim 5, wherein the ADNF I polypeptide comprises all D-amino acids.

7. The ADNF I polypeptide of claim 1, wherein the ADNF I polypeptide is selected from the group consisting of: Val-Leu-Gly-Gly-Gly- Ser-Ala-Leu-Leu-Arg-Ser-Ile-Pro-Ala (SEQ ID NO:14); 
Val-Glu-Glu-Gly-Ile-Val-Leu-Gly-Gly-Gly-Ser-Ala-Leu-Leu-Arg-Ser-Ile-Pro-
Ala (SEQ ID NO:15);
 Leu-Gly-Gly-Gly- Ser-Ala-Leu-Leu-Arg-Ser-Ile-Pro-Ala (SEQ ID NO:16); Gly-Gly-Gly-Ser-Ala-Leu-Leu-Arg-Ser-Ile-Pro-Ala (SEQ ID NO:17); Gly-Gly-Ser-Ala-Leu-Leu-Arg-Ser-Ile-Pro-Ala (SEQ ID NO:18); and

Gly- Ser-Ala-Leu-Leu-Arg-Ser-Ile-Pro-Ala (SEQ ID NO:19).

8. The ADNF I polypeptide of claim 1, wherein the ADNF I polypeptide comprises up to 20 amino acids at each of an N-terminus and a C-terminus of the active core site.

9. The ADNF I polypeptide of claim 8, wherein both N-terminal and C-terminal amino acids of the ADNF I polypeptide are D-amino acids.

10. An Activity Dependent Neurotrophic Factor III (ADNF III) polypeptide, the ADNF III polypeptide comprising an active core site having the following amino acid sequence: Asn-Ala-Pro-Val-Ser-Ile-Pro-Gln (SEQ ID NO:2), wherein the active core site comprises at least one D-amino acid.

11. The ADNF III polypeptide of claim 10, wherein either an N-terminal amino acid or an C-terminal amino acid of the active core site is a D-amino acid.

12. The ADNF III polypeptide of claim 10, wherein both N-terminal and C-terminal amino acids of the active core site are D-amino acids.

13. The ADNF III polypeptide of claim 10, wherein the active core site comprises all D-amino acids.

14. The ADNF III polypeptide of claim 10, wherein the ADNF III polypeptide is Asn-Ala-Pro-Val-Ser-Ile-Pro-Gln (SEQ ID NO:2).

15. The ADNF III polypeptide of claim 14, wherein the ADNF III polypeptide comprises all D-amino acids.

16. The ADNF III polypeptide of claim 10, wherein the ADNF III polypeptide is selected from the group consisting of: Gly-Gly-Asn-Ala-Pro-Val-Ser-Ile-Pro-Gln (SEQ ID NO:20); Leu-Gly-Gly-Asn-Ala-Pro-Val-Ser-Ile-Pro-Gln-Gln-Ser (SEQ ID NO:21); 
Leu-Gly-Leu-Gly-Gly-Asn-Ala-Pro-Val-Ser-Ile-Pro-Gln-Gln-Ser (SEQ ID
NO:22);
 and

Ser-Val-Arg-Leu-Gly-Leu-Gly-Gly-Asn-Ala-Pro-Val-Ser-Ile-Pro-Gln-Gln-Ser
(SEQ ID NO:23).

17. The ADNF III polypeptide of claim 10, wherein the ADNF III polypeptide comprises up to 20 amino acids at each of an N-terminus and an C-terminus of the active core site.

18. The ADNF III polypeptide of claim 17, wherein both N-terminal and C-terminal amino acids of the ADNF III polypeptide are D-amino acids.

19. A pharmaceutical composition comprising a pharmaceutically acceptable excipient and an Activity Dependent Neurotrophic Factor (ADNF) polypeptide, wherein the ADNF polypeptide is a member selected from the group consisting of:
(a) an ADNF I polypeptide comprising an active core site having the following amino acid sequence: Ser-Ala-Leu-Leu-Arg-Ser-Ile-Pro-Ala (SEQ ID NO:1);
(b) an ADNF III polypeptide comprising an active core site having the following amino acid sequence: Asn-Ala-Pro-Val-Ser-Ile-Pro-Gln (SEQ ID NO:2); ; and
(c) a mixture of the ADNF I polypeptide of part (a) and the ADNF III polypeptide of part (b);
wherein at least one of the ADNF I polypeptide and the ADNF III polypeptide comprises an active core site comprising at least one D-amino acid.

20. The pharmaceutical composition of claim 19, wherein the ADNF polypeptide is an ADNF I polypeptide and wherein the active core site of the ADNF I polypeptide comprises at least one D-amino acid.

21. The pharmaceutical composition of claim 20, wherein both N-terminal and C-terminal amino acids of the active core site of the ADNF I polypeptide are D-amino acids.

22. The pharmaceutical composition of claim 20, wherein the active core site of the ADNF I polypeptide comprises all D-amino acids.

23. The pharmaceutical composition of claim 20, wherein the ADNF I polypeptide is Ser-Ala-Leu-Leu-Arg-Ser-Ile-Pro-Ala (SEQ ID NO:1).

24. The pharmaceutical composition of claim 23, wherein the ADNF I polypeptide comprises all D-amino acids.

25. The pharmaceutical composition of claim 19, wherein the ADNF polypeptide is an ADNF III polypeptide and wherein the active core site of the ADNF III polypeptide comprises at least one D-amino acid.

26. The pharmaceutical composition of claim 25, wherein both N-terminal and C-terminal amino acids of the active core site of the ADNF III polypeptide are D-amino acids.

27. The pharmaceutical composition of claim 25, wherein the active core site of the ADNF III polypeptide comprises all D-amino acids.

28. The pharmaceutical composition of claim 25, wherein the ADNF III polypeptide is Asn-Ala-Pro-Val-Ser-Ile-Pro-Gln (SEQ ID NO:2).

29. The pharmaceutical composition of claim 28, wherein the ADNF III polypeptide comprises all D-amino acids.

30. The pharmaceutical composition of claim 19, wherein the ADNF polypeptide is a mixture of an ADNF I polypeptide of part (a) and an ADNF III polypeptide of part (b) and wherein at least one of the ADNF I polypeptide and the ADNF III polypeptide comprises an active core site comprising at least one D-amino acid.

31. The pharmaceutical composition of claim 30, wherein both N-terminal and C-terminal amino acids of the active core site of the ADNF I polypeptide are D-amino acids, and wherein both N-terminal and C-terminal amino acids of the active core site of the ADNF III polypeptide are D-amino acids.

32. The pharmaceutical composition of claim 30, wherein the active core site of the ADNF I polypeptide comprises all D-amino acids, and wherein the active core site of the ADNF III polypeptide comprises all D-amino acids.

33. The pharmaceutical composition of claim 30, wherein the ADNF I polypeptide is Ser-Ala-Leu-Leu-Arg-Ser-Ile-Pro-Ala (SEQ ID NO:1) and wherein the ADNF III polypeptide is Asn-Ala-Pro-Val-Ser-Ile-Pro-Gln (SEQ ID NO:2).

34. The pharmaceutical composition of claim 33, wherein the ADNF I polypeptide comprises all D-amino acids and wherein the ADNF III polypeptide comprises all D-amino acids.

35. The pharmaceutical composition of claim 30, wherein the ADNF I polypeptide comprises all D-amino acids and wherein the ADNF III polypeptide comprises all L-amino acids.

36. The pharmaceutical composition of claim 30, wherein the ADNF I polypeptide comprises all L-amino acids, and wherein the ADNF III polypeptide comprises all D-amino acids.

37. The pharmaceutical composition of claim 19, wherein the composition is formulated for intranasal, intraperitoneal, subcutaneous, gavage, sublingual, intravenous, or oral administration.

38. The pharmaceutical composition of one of claims 19, 22, 27 or 32,wherein the composition is formulated for oral administration.

39. Pharmaceutical composition, in particular as defined in at least one of claims 19 to 38, for use as a medicament in a treatment of reducing neuronal cell death, comprising contacting the neuronal cells with an Activity Dependent Neurotrophic Factor (ADNF) polypeptide in an amount sufficient to prevent neuronal cell death, wherein the ADNF polypeptide is a member selected from the group consisting of:
(a) an ADNF I polypeptide comprising an active core site having the following amino acid sequence: Ser-Ala-Leu-Leu-Arg-Ser-Ile-Pro-Ala (SEQ ID NO: 1);
(b) an ADNF III polypeptide having the following amino acid sequence: Asn-Ala-Pro-Val-Ser-Ile-Pro-Gln (SEQ ID NO: 2); and
(c) a mixture of the ADNF I polypeptide of part (a) and the ADNF III polypeptide of part (b);
wherein at least one of the ADNF I polypeptide and the ADNF III polypeptide comprises an active core site comprising at least one D-amino acid.

40. The pharmaceutical composition for use as a medicament in a treatment according to claim 39, wherein the neuronal cells are selected from the group consisting of spinal cord neurons, hippocampal neurons, cerebral cortical neurons and cholinergic neurons.

41. The pharmaceutical composition for use as a medicament in a treatment according to claim 39, wherein the neuronal cell death is in a patient infected with immunodeficiency virus, in particular wherein the immunodeficiency virus is human immunodeficiency virus.

42. The pharmaceutical composition for use as a medicament in a treatment according to claim 39, wherein the neuronal cell death is associated with excito-toxicity induced by N-methyl-D-aspartate stimulation.

43. The pharmaceutical composition for use as a medicament in a treatment according to claim 39, wherein the neuronal cell death is induced by the beta-amyloid peptide in a patient afflicted with Alzheimer's disease.

44. The pharmaceutical composition for use as a medicament in a treatment according to claim 39, wherein the neuronal cell death is induced by cholinergic blockade in a patient afflicted with Alzheimer's disease, the cholinergic blockade resulting in learning impairment.

45. Pharmaceutical composition, in particular as defined in at least one of claims 19 to 38, for use as a medicament in a treatment of oxidative stress in a patient, comprising administering to the patient an Activity Dependent Neurotrophic Factor (ADNF) polypeptide in an amount sufficient to reduce oxidative stress, wherein the ADNF polypeptide is a member selected from the group consisting of:
(a) an ADNF I polypeptide comprising an active core site having the following amino acid sequence: Ser-Ala-Leu-Leu-Arg-Ser-Ile-Pro-Ala (SEQ ID NO: 1);
(b) an ADNF III polypeptide having the following amino acid sequence: Asn-Ala-Pro-Val-Ser-Ile-Pro-Gln (SEQ ID NO: 2); and
(c) a mixture of the ADNF I polypeptide of part (a) and the ADNF III polypeptide of part (b);
wherein at least one of the ADNF I polypeptide and the ADNF III polypeptide comprises an active core site comprising at least one D-amino acid.

46. Pharmaceutical composition, in particular as defined in at least one of claims 19 to 38, for use as a medicament in a treatment of reducing a condition associated with fetal alcohol syndrome in a subject who is exposed to alcohol *in utero*, comprising administering to the subject an Activity Dependent Neurotrophic Factor (ADNF) polypeptide in an amount sufficient to reduce the condition associated with fetal alcohol syndrome, wherein the ADNF polypeptide is a member selected from the group consisting of:
(a) an ADNF I polypeptide comprising an active core site having the following amino acid sequence: Ser-Ala-Leu-Leu-Arg-Ser-Ile-Pro-Ala (SEQ ID NO: 1);
(b) an ADNF III polypeptide having the following amino acid sequence: Asn-Ala-Pro-Val-Ser-Ile-Pro-Gln (SEQ ID NO: 2); and
(c) a mixture of the ADNF I polypeptide of part (a) and the ADNF III polypeptide of part (b);
wherein at least one of the ADNF I polypeptide and the ADNF III polypeptide comprises an active core site comprising at least one D-amino acid.

47. The pharmaceutical composition for use as a medicament in a treatment according to claim 46, wherein the condition is selected from the group consisting of: a decreased body weight of a subject, a decreased brain weight of the subject, a decreased level of VIP mRNA of a subject; and death of a subject *in utero*.

48. Use of the pharmaceutical composition, in particular as defined in at least one of claims 19 to 38, for the manufacture of a medicament for reducing neuronal cell death according to at least one of claims 39 to 44.

49. Use of the pharmaceutical composition, in particular as defined in at least one of claims 19 to 38, for the manufacture of a medicament for treatment of oxidative stress in a patient according to claim 45.

50. Use of the pharmaceutical composition, in particular as defined in at least one of claims 19 to 38, for the manufacture of a medicament for treatment of reducing a condition associated with fetal alcohol syndrome in a subject who is exposed to alcohol *in utero* according to at least one of claims 46 to 47.

## Patentansprüche

1. Aktivitätsabhängiger neurotrophischer Faktor I -Polypeptid (ADNF I), bei dem das ADNF I-Polypeptid eine aktive Kernstelle mit folgender Aminosäurensequenz umfasst:
Ser-Ala-Leu-Leu-Arg-Ser-Ile-Pro-Ala (SEQ ID NR: 1),
bei dem die aktive Kernstelle mindestens eine D-Aminosäure umfasst.

2. ADNF I-Polypeptid nach Anspruch 1, bei dem entweder eine N-terminale Aminosäure oder eine C-terminale Aminosäure der aktiven Kernstelle eine D-Aminosäure ist.

3. ADNF I-Polypeptid nach Anspruch 1, bei dem sowohl die N-terminalen als auch die C-terminalen Aminosäuren der aktiven Kernstelle D-Aminosäuren sind.

4. ADNF I-Polypeptid nach Anspruch 1, bei dem die aktive Kernstelle alle D-Aminosäuren umfasst.

5. ADNF I-Polypeptid nach Anspruch 1, bei dem das ADNF I-Polypeptid Ser-Ala-Leu-Leu-Arg-Ser-Ile-Pro-Ala (SEQ ID NR: 1) ist.

6. ADNF I-Polypeptid nach Anspruch 5, bei dem das ADNF I-Polypeptid alle D-Aminosäuren umfasst.

7. ADNF I-Polypeptid nach Anspruch 1, bei dem das ADNF I-Polypeptid aus wie folgt zusammengesetzter Gruppe ausgewählt wird: Val-Leu-Gly-Gly-Gly-Ser-Ala-Leu-Leu-Arg-Ser-Ile-Pro-Ala (SEQ ID NR. 14); 
Val-Glu-Glu-Gly-Ile-Val-Leu-Gly-Gly-Gly-Ser-Ala-Leu-Leu-Arg-Ser-Ile-Pro-Ala
(SEQ ID NR. 15);
 Leu-Gly-Gly-Gly-Ser-Ala-Leu-Leu-Arg-Ser-Ile-Pro-Ala (SEQ ID NR: 16); Gly-Gly-Gly-Ser-Ala-Leu-Leu-Arg-Ser-Ile-Pro-Ala (SEQ ID NR: 17); Gly-Gly-Ser-Ala-Leu-Leu-Arg-Ser-Ile-Pro-Ala (SEQ ID NR: 18); und

Gly-Ser-Ala-Leu-Leu-Arg-Ser-Ile-Pro-Ala (SEQ ID NR: 19).

8. ADNF I-Polypeptid nach Anspruch 1, bei dem das ADNF I-Polypeptid bis zu 20 Aminosäuren an jedem eines N-Terminus und C-Terminus der aktiven Kernstelle umfasst.

9. ADNF I-Polypeptid nach Anspruch 8, bei dem sowohl N-terminale als auch C-terminale Aminosäuren des ADNF I-Polypeptids D-Aminosäuren sind.

10. Aktivitätsabhängiger neurotrophischer Faktor III-Polypeptid (ADNF III), bei dem das ADNF III-Polypeptid eine aktive Kernstelle mit folgender Aminosäurensequenz umfasst: Asn-Ala-Pro-Val-Ser-Ile-Pro-Gln (SEQ ID NR: 2), bei dem die aktive Kernstelle mindestens eine D-Aminosäure umfasst.

11. ADNF III-Polypeptid nach Anspruch 10, bei dem entweder eine N-terminale oder eine C-terminale Aminosäure der aktiven Kernstelle eine D-Aminosäure ist.

12. ADNF III-Polypeptid nach Anspruch 10, bei dem sowohl die N-terminalen als auch die C-terminalen Aminosäuren der aktiven Kernstelle D-Aminosäuren sind.

13. ADNF III-Polypeptid nach Anspruch 10, bei dem die aktive Kernstelle alle D-Aminosäuren umfasst.

14. ADNF III-Polypeptid nach Anspruch 10, bei dem das ADNF III-Polypeptid Asn-Ala-Pro-Val-Ser-Ile-Pro-Gln (SEQ ID NR:2) ist.

15. ADNF III-Polypeptid nach Anspruch 14, bei dem das ADNF III-Polypeptid alle D-Aminosäuren umfasst.

16. ADNF III-Polypeptid nach Anspruch 10, bei dem das ADNF III-Polypeptid aus wie folgt zusammengesetzter Gruppe ausgewählt wird: Gly-Gly-Asn-Ala-Pro-Val-Ser-Ile-Pro-Gln (SEQ ID NR: 20); Leu-Gly-Gly-Asn-Ala-Pro-Val-Ser-Ile-Pro-Gln-Gln-Ser (SEQ ID NR: 21); Leu-Gly-Leu-Gly-Gly-Asn-Ala-Pro-Val-Ser-Ile-Pro-Gln-Gln-Ser (SEQ ID NR: 22); und

Ser-Val-Arg-Leu-Gly-Leu-Gly-Gly-Asn-Ala-Pro-Val-Ser-Ile-Pro-Gln-Gln-Ser (SEQ
ID NR: 23).

17. ADNF III-Polypeptid nach Anspruch 10, bei dem das ADNF III-Polypeptid bis zu 20 Aminosäuren an jedem eines N-Terminus und C-Terminus der aktiven Kernstelle umfasst.

18. ADNF III-Polypeptid nach Anspruch 17, bei dem sowohl die N-terminalen als auch die C-terminalen Aminosäuren des ADNF III-Polypeptids D-Aminosäuren sind.

19. Pharmazeutische Zusammensetzung mit einem pharmazeutisch annehmbaren Trägerstoff und einem aktivitätsabhängiger neurotrophischer Faktor (ADNF)-Polypeptid, bei der das ADNF-Polypeptid aus wie folgt zusammengesetzter Gruppe ausgewählt wird:
(a) ADNF I-Polypeptid mit einer aktiven Kernstelle, die folgende Aminosäurensequenz aufweist: Ser-Ala-Leu-Leu-Arg-Ser-Ile-Pro-Ala (SEQ ID NR: 1);
(b) ADNF III-Polypeptid mit einer aktiven Kernstelle, die folgende Aminosäurensequenz aufweist: Asn-Ala-Pro-Val-Ser-Ile-Pro-Gln (SEQ ID NR. 2); und
(c) Mischung des ADNF I-Polypeptids aus Punkt (a) und des ADNF III-Polypeptids aus Punkt (b);
bei der mindestens eins der ADNF I- und ADNF III-Polypeptide eine aktive Kernstelle mit mindestens einer D-Aminosäure umfasst.

20. Pharmazeutische Zusammensetzung nach Anspruch 19, bei der das ADNF-Polypeptid ein ADNF I-Polypeptid ist und die aktive Kernstelle des ADNF I-Polypeptids mindestens eine D-Aminosäure umfasst.

21. Pharmazeutische Zusammensetzung nach Anspruch 20, bei der sowohl die N-terminalen als auch die C-terminalen Aminosäuren der aktiven Kernstelle des ADNF I-Polypeptids D-Aminosäuren sind.

22. Pharmazeutische Zusammensetzung nach Anspruch 20, bei der die aktive Kernstelle des ADNF I-Polypeptids alle D-Aminosäuren umfasst.

23. Pharmazeutische Zusammensetzung nach Anspruch 20, bei der das ADNF I-Polypeptid Ser-Ala-Leu-Leu-Arg-Ser-Ile-Pro-Ala (SEQ ID NR: 1) ist.

24. Pharmazeutische Zusammensetzung nach Anspruch 23, bei der das ADNF I-Polypeptid alle D-Aminosäuren umfasst.

25. Pharmazeutische Zusammensetzung nach Anspruch 19, bei der das ADNF-Polypeptid ein ADNF III-Polypeptid ist und die aktive Kernstelle des ADNF III-Polypeptids mindestens eine D-Aminosäure umfasst.

26. Pharmazeutische Zusammensetzung nach Anspruch 25, bei der sowohl die N-terminalen als auch die C-terminalen Aminosäuren der aktiven Kernstelle des ADNF III-Polypeptids D-Aminosäuren sind.

27. Pharmazeutische Zusammensetzung nach Anspruch 25, bei der die aktive Kernstelle des ADNF III-Polypeptids alle D-Aminosäuren umfasst.

28. Pharmazeutische Zusammensetzung nach Anspruch 25, bei der das ADNF III-Polypeptid Asn-Ala-Pro-Val-Ser-Ile-Pro-Gln (SEQ ID NR: 2) ist.

29. Pharmazeutische Zusammensetzung nach Anspruch 28, bei der das ADNF III-Polypeptid alle D-Aminosäuren umfasst.

30. Pharmazeutische Zusammensetzung nach Anspruch 19, bei der das ADNF-Polypeptid eine Mischung eines ADNF I-Polypeptids aus Punkt (a) und eines ADNF III-Polypeptids aus Punkt (b) ist und mindestens eins der ADNF I und ADNF III-Polypeptide eine aktive Kernstelle mit mindestens einer D-Aminosäure umfasst.

31. Pharmazeutische Zusammensetzung nach Anspruch 30, bei der sowohl die N-terminalen als auch die C-terminalen Aminosäuren der aktiven Kernstelle des ADNF I-Polypeptids D-Aminosäuren sind und sowohl die N-terminalen als auch die C-terminalen Aminosäuren der aktiven Kernstelle des ADNF III-Polypeptids D-Aminosäuren sind.

32. Pharmazeutische Zusammensetzung nach Anspruch 30, bei der die aktive Kernstelle des ADNF I-Polypeptids alle D-Aminosäuren umfasst und die aktive Kernstelle des ADNF III-Polypeptids alle D-Aminosäuren umfasst.

33. Pharmazeutische Zusammensetzung nach Anspruch 30, bei der das ADNF I-Polypeptid Ser-Ala-Leu-Leu-Arg-Ser-Ile-Pro-Ala (SEQ ID NR1) ist und das ADNF III-Polypeptid Asn-Ala-Pro-Val-Ser-Ile-Pro-Gln (SEQ ID NR: 2) ist.

34. Pharmazeutische Zusammensetzung nach Anspruch 33, bei der das ADNF I-Polypeptid alle D-Aminosäuren umfasst und das ADNF III-Polypeptid alle D-Aminosäuren umfasst.

35. Pharmazeutische Zusammensetzung nach Anspruch 30, bei der das ADNF I-Polypeptid alle D-Aminosäuren umfasst und das ADNF III-Polypeptid alle L-Aminosäuren umfasst.

36. Pharmazeutische Zusammensetzung nach Anspruch 30, bei der das ADNF I-Polypeptid alle L-Aminosäuren umfasst und das ADNF III-Polypeptid alle D-Aminosäuren umfasst.

37. Pharmazeutische Zusammensetzung nach Anspruch 19, wobei die Zusammensetzung für eine intranasale, intraperitoneale, subkutane, sublinguale, intravenöse, orale bzw. für eine Verabreichung über eine Sonde zubereitet wird.

38. Pharmazeutische Zusammensetzung nach einem der Ansprüche 19, 22, 27 oder 32, wobei die Zusammensetzung für eine orale Verabreichung zubereitet wird.

39. Pharmazeutische Zusammensetzung, insbesondere wie durch mindestens einen der Ansprüche 19 bis 38 definiert, zur Anwendung als Medikament bei einer Behandlung zur Reduzierung neuronalen Zelltods, die das Inkontaktbringen der Nervenzellen mit einem aktivitätsabhängiger neurotrophischer Faktor (ADNF) Polypeptid in ausreichender Menge einschließt, um neuronalen Zelltod zu verhindern, bei der das ADNF-Polypeptid aus wie folgt zusammengesetzter Gruppe ausgewählt wird:
(a) ADNF I-Polypeptid mit einer aktiven Kernstelle, die folgende Aminosäurenseauenz aufweist: Ser-Ala-Leu-Leu-Arg-Ser-Ile-Pro-Ala (SEQ ID NR: 1);
(b) ADNF III-Polypeptid mit folgender Aminosäurensequenz: Asn-Ala-Pro-Val-Ser-Ile-Pro-Gln (SEQ ID NR: 2); und
(c) Mischung des ADNF I-Polypeptids aus Punkt (a) und des ADNF III-Polypeptids aus Punkt (b);
bei der mindestens eins der ADNF I- und ADNF III-Polypeptide eine aktive Kernstelle mit mindestens einer D-Aminosäure umfasst.

40. Pharmazeutische Zusammensetzung zur Anwendung als Medikament bei einer Behandlung nach Anspruch 39, wobei die Nervenzellen aus der aus Rückenmark-, Hippokampus-, Großhirnrinden- und cholinergischen Nervenzellen bestehenden Gruppe ausgewählt werden.

41. Pharmazeutische Zusammensetzung zur Anwendung als Medikament bei einer Behandlung nach Anspruch 39, wobei der neuronale Zelltod bei einem mit Immundefizienz hervorrufenden Virus infizierten Patienten auftritt, insbesondere wenn das Immundefizienz hervorrufende Virus das HIV-Virus ist.

42. Pharmazeutische Zusammensetzung zur Anwendung als Medikament bei einer Behandlung nach Anspruch 39, wobei der neuronale Zelltod mit einer Exzitotoxizität in Verbindung steht, die durch die Stimulation mit N-Methyl-D-Aspartat induziert wird.

43. Pharmazeutische Zusammensetzung zur Anwendung als Medikament bei einer Behandlung nach Anspruch 39, wobei der neuronale Zelltod bei einem unter Alzheimer leidenden Patienten durch das Beta-Amyloid-Peptid induziert wird.

44. Pharmazeutische Zusammensetzung zur Anwendung als Medikament bei einer Behandlung nach Anspruch 39, wobei der neuronale Zelltod bei einem unter Alzheimer leidenden Patienten durch cholinergische Blockade induziert wird und diese zu Lernschwierigkeiten führt.

45. Pharmazeutische Zusammensetzung, insbesondere wie durch mindestens einen der Ansprüche 19 bis 38 definiert, zur Anwendung als Medikament bei einer Behandlung oxidativen Stresses bei einem Patienten, die das Verabreichen eines aktivitätsabhängiger neurotrophischer Faktor (ADNF)-Polypeptids in ausreichender Menge umfasst, um oxidativen Stress zu reduzieren, bei der das ADNF-Polypeptid aus wie folgt zusammengesetzter Gruppe ausgewählt wird:
(a) ADNF I-Polypeptid mit einer aktiven Kemstelle, die folgende Aminosäurensequenz aufweist: Ser-Ala-Leu-Leu-Arg-Ser-Ile-Pro-Ala (SEQ ID NR: 1);
(b) ADNF III-Polypeptid mit folgender Aminosäurensequenz: Asn-Ala-Pro-Val-Ser-Ile-Pro-Gln (SEQ ID NR: 2); und
(c) Mischung des ADNF I-Polypeptids aus Punkt (a) und des ADNF III-Polypeptids aus Punkt (b);
bei der mindestens eins der ADNF I- und ADNF III-Polypeptide eine aktive Kernstelle mit mindestens einer D-Aminosäure umfasst.

46. Pharmazeutische Zusammensetzung, insbesondere wie durch mindestens einen der Ansprüche 19 bis 38 definiert, zur Anwendung als Medikament bei einer Behandlung eines mit dem fetalen Alkoholsyndrom assoziierten Zustands bei einem Subjekt, das *in utero* Alkohol ausgesetzt ist, wobei die Behandlung das Verabreichen eines aktivitätsabhängiger neurotrophischer Faktor (ADNF)-Polypeptids an das Subjekt in ausreichender Menge umfasst, um den mit dem fetalen Alkoholsyndrom assoziierten Zustand zu reduzieren, wobei das ADNF-Polypeptid aus wie folgt zusammengesetzter Gruppe ausgewählt wird:
(a) ADNF I-Polypeptid mit einer aktiven Kernstelle, die folgende Aminosäurensequenz aufweist: Ser-Ala-Leu-Leu-Arg-Ser-Ile-Pro-Ala (SEQ ID NR: 1);
(b) ADNF III-Polypeptid mit folgender Aminosäurensequenz: Asn-Ala-Pro-Val-Ser-Ile-Pro-Gln (SEQ ID NR: 2); und
(c) Mischung des ADNF I-Polypeptids aus Punkt (a) und des ADNF III-Polypeptids aus Punkt (b);
bei der mindestens eins der ADNF I- und ADNF III-Polypeptide eine aktive Kernstelle mit mindestens einer D-Aminosäure umfasst.

47. Pharmazeutische Zusammensetzung zur Anwendung als Medikament bei einer Behandlung nach Anspruch 46, bei der der Zustand aus wie folgt zusammengesetzter Gruppe ausgewählt wird: verringertes Körpergewicht eines Subjekts, verringertes Gewicht des Gehirns des Subjekts, ein verringertes VIP mRNA-Level eines Subjekts und Tod eines Subjekts *in utero.*

48. Anwendung der pharmazeutischen Zusammensetzung, insbesondere wie durch mindestens einen der Ansprüche 19 bis 38 definiert, zur Herstellung eines Medikaments zur Reduzierung neuronalen Zelltods nach mindestens einem der Ansprüche 39 bis 44.

49. Anwendung der pharmazeutischen Zusammensetzung, insbesondere wie durch mindestens einen der Ansprüche 19 bis 38 definiert, zur Herstellung eines Medikaments für die Behandlung oxidativen Stresses bei einem Patienten nach Anspruch 45.

50. Anwendung der pharmazeutischen Zusammensetzung, insbesondere wie durch mindestens einen der Ansprüche 19 bis 38 definiert, zur Herstellung eines Medikaments zur Reduzierung eines mit dem fetalen Alkoholsyndrom assoziierten Zustands bei einem *in utero* Alkohol ausgesetzten Subjekt nach mindestens einem der Ansprüche 46 bis 47.

## Revendications

1. Polypeptide du facteur neurotrophique dépendant de l'activité I (ADNF I), le polypeptide ADNF I comprenant un site central actif ayant la séquence d'acides aminés suivante : Ser-Ala-Leu-Leu-Arg-Ser-Ile-Pro-Ala (N° d'ID de SÉQ : 1), dans lequel le site central actif comprend au moins un acide aminé D.

2. Polypeptide ADNF I selon la revendication 1, dans lequel soit un acide aminé N-terminal soit un acide aminé C-terminal du site central actif est un acide aminé D.

3. Polypeptide ADNF I selon la revendication 1, dans lequel les acides aminés N-terminal et C-terminal du site central actif sont des acides aminés D.

4. Polypeptide ADNF I selon la revendication 1, dans lequel le site central actif comprend tous les acides aminés D.

5. Polypeptide ADNF I selon la revendication 1, dans lequel le polypeptide ADNF I est Ser-Ala-Leu-Leu-Arg-Ser-Ile-Pro-Ala (N° d'ID de SÉQ : 1).

6. Polypeptide ADNF I selon la revendication 5, dans lequel le polypeptide ADNF I comprend tous les acides aminés D.

7. Polypeptide ADNF I selon la revendication 1, dans lequel le polypeptide ADNF I est sélectionné parmi le groupe composé de : Val-Leu-Gly-Gly-Gly-Ser-Ala-Leu-Leu-Arg-Ser-Ile-Pro-Ala (N° d'ÌD de SÉQ : 14) ; 
Val-Glu-Glu-Gly-Ile-Val-Leu-Gly-Gly-Gly-Ser-Ala-Leu-Leu-Arg-Ser-Ile-Pro-Ala (N°
d'ID de SÉQ : 15) ;
 Leu-Gly-Gly-Gly-Ser-Ala-Leu-Leu-Arg-Ser-Ile-Pro-Ala (N° d'ID de SÉQ : 16) ; Gly-Gly-Gly-Ser-Ala-Leu-Leu-Arg-Ser-Ile-Pro-Ala (N° d'ID de SÉQ : 17) ; Gly-Gly-Ser-Ala-Leu-Leu-Arg-Ser-Ile-Pro-Ala (N° d'ID de SÉQ : 18) ; et

Gly-Ser-Ala-Leu-Leu-Arg-Ser-Ile-Pro-Ala (N° d'ID de SÉQ : 19).

8. Polypeptide ADNF I selon la revendication 1, dans lequel le polypeptide ADNF I comprend jusqu'à 20 acides aminés sur chacun d'un N-terminal et C-terminal du site central actif.

9. Polypeptide ADNF I selon la revendication 8, dans lequel les acides aminés N-terminal et C-terminal du polypeptide ADNF I sont des acides aminés D.

10. Polypeptide du Facteur Neurotrophique Dépendant de l'Activité III (ADNF III), le polypeptide ADNF III comprenant un site central actif ayant la séquence d'acides aminés suivante : Asn-Ala-Pro-Val-Ser-Ile-Pro-Gln (N° d'ID de SÉQ : 2), dans lequel le site central actif comprend au moins un acide aminé D.

11. Polypeptide ADNF III selon la revendication 10, dans lequel soit un acide aminé N-terminal soit un acide aminé C-terminal du site central actif est un acide aminé D.

12. Polypeptide ADNF III selon la revendication 10, dans lequel les acides aminés N-terminal et C-terminal du site central actif sont des acides aminés D.

13. Polypeptide ADNF III selon la revendication 10, dans lequel le site central actif comprend tous les acides aminés D.

14. Polypeptide ADNF III selon la revendication 10, dans lequel le polypeptide ADNF III est Asn-Ala-Pro-Val-Ser-Ile-Pro-Gln (N° d'ID de SÉQ :2).

15. Polypeptide ADNF III selon la revendication 14, dans lequel le polypeptide ADNF III comprend tous les acides aminés D.

16. Polypeptide ADNF III selon la revendication 10, dans lequel le polypeptide ADNF III est sélectionné parmi le groupe composé de : Gly-Gly-Asn-Ala-Pro-Val-Ser-Ile-Pro-Gln (N° d'ID de SÉQ : 20) ; Leu-Gly-Gly-Asn-Ala-Pro-Val-Ser-Ile-Pro-Gln-Gln-Ser (N° d'ID de SÉQ : 21) ; 
Leu-Gly-Leu-Gly-Gly-Asn-Ala-Pro-Val-Ser-Ile-Pro-Gln-Gln-Ser (N° d'ID de SÉQ :
22) ;
 et

Ser-Val-Arg-Leu-Gly-Leu-Gly-Gly-Asn-Ala-Pro-Val-Ser-Ile-Pro-Gln-Gln-Ser (N°
d'ID de SÉQ : 23).

17. Polypeptide ADNF III selon la revendication 10, dans lequel le polypeptide ADNF III comprend jusqu'à 20 acides aminés sur chacun d'un N-terminal et C-terminal du site central actif.

18. Polypeptide ADNF III selon la revendication 17, dans lequel les acides aminés N-terminal et C-terminal du polypeptide ADNF III sont des acides aminés D.

19. Composition pharmaceutique comprenant un excipient pharmaceutiquement acceptable et un polypeptide du Facteur Neurotrophique Dépendant de l'Activité (ADNF), dans laquelle le polypeptide ADNF est un élément sélectionné parmi le groupe composé de :
(a) un polypeptide ADNF I comprenant un site central actif ayant la séquence d'acides aminés suivante : Ser-Ala-Leu-Leu-Arg-Ser-Ile-Pro-Ala (N° d'ID de SÉQ : 1) ;
(b) un polypeptide ADNF III comprenant un site central actif ayant la séquence d'acides aminés suivante : Asn-Ala-Pro-Val-Ser-Ile-Pro-Gln (N° d'ID de SÉQ : 2) ; et
(c) un mélange du polypeptide ADNF I du point (a) et du polypeptide ADNF III du point (b) ;
dans laquelle au moins l'un des polypeptides ADNF I et ADNF III comprend un site central actif comprenant au moins un acide aminé D.

20. Composition pharmaceutique selon la revendication 19, dans laquelle le polypeptide ADNF est un polypeptide ADNF I et dans laquelle le site central actif du polypeptide ADNF I comprend au moins un acide aminé D.

21. Composition pharmaceutique selon la revendication 20, dans laquelle les acides aminés N-terminal et C-terminal du site central actif du polypeptide ADNF I sont des acides aminés D.

22. Composition pharmaceutique selon la revendication 20, dans laquelle le site central actif du polypeptide ADNF I comprend tous les acides aminés D.

23. Composition pharmaceutique selon la revendication 20, dans laquelle le polypeptide ADNF I est Ser-Ala-Leu-Leu-Arg-Ser-Ile-Pro-Ala (N° d'ID de SÉQ : 1).

24. Composition pharmaceutique selon la revendication 23, dans laquelle le polypeptide ADNF I comprend tous les acides aminés D.

25. Composition pharmaceutique selon la revendication 19, dans laquelle le polypeptide ADNF est un polypeptide ADNF III et dans laquelle le site central actif du polypeptide ADNF III comprend au moins un acide aminé D.

26. Composition pharmaceutique selon la revendication 25, dans laquelle les acides aminés N-terminal et C-terminal du site central actif du polypeptide ADNF III sont des acides aminés D.

27. Composition pharmaceutique selon la revendication 25, dans laquelle le site central actif du polypeptide ADNF III comprend tous les acides aminés D.

28. Composition pharmaceutique selon la revendication 25, dans laquelle le polypeptide ADNF III est Asn-Ala-Pro-Val-Ser-Ile-Pro-Gln (N° d'ID de SÉQ : 2).

29. Composition pharmaceutique selon la revendication 28, dans laquelle le polypeptide ADNF III comprend tous les acides aminés D.

30. Composition pharmaceutique selon la revendication 19, dans laquelle le polypeptide ADNF est un mélange d'un polypeptide ADNF I du point (a) et d'un polypeptide ADNF III du point (b) et dans laquelle au moins l'un des polypeptides ADNF I et ADNF III comprend un site central actif comprenant au moins un acide aminé D.

31. Composition pharmaceutique selon la revendication 30, dans laquelle les acides aminés N-terminal et C-terminal du site central actif du polypeptide ADNF I sont des acides aminés D, et dans laquelle les acides aminés N-terminal et C-terminal du site central actif du polypeptide ADNF III sont des acides aminés D.

32. Composition pharmaceutique selon la revendication 30, dans laquelle le site central actif du polypeptide ADNF I comprend tous les acides aminés D, et dans laquelle le site central actif du polypeptide ADNF III comprend tous les acides aminés D.

33. Composition pharmaceutique selon la revendication 30, dans laquelle le polypeptide ADNF I est Ser-Ala-Leu-Leu-Arg-Ser-Ile-Pro-Ala (N° d'ID de SÉQ : 1) et dans laquelle le polypeptide ADNF III est Asn-Ala-Pro-Val-Ser-Ile-Pro-Gln (N° d'ID de SÉQ : 2).

34. Composition pharmaceutique selon la revendication 33, dans laquelle le polypeptide ADNF I comprend tous les acides aminés D et dans laquelle le polypeptide ADNF III comprend tous les acides aminés D.

35. Composition pharmaceutique selon la revendication 30, dans laquelle le polypeptide ADNF I comprend tous les acides aminés D et dans laquelle le polypeptide ADNF III comprend tous les acides aminés L.

36. Composition pharmaceutique selon la revendication 30, dans laquelle le polypeptide ADNF I comprend tous les acides aminés L, et dans laquelle le polypeptide ADNF III comprend tous les acides aminés D.

37. Composition pharmaceutique selon la revendication 19, dans laquelle la composition est formulée pour une administration intranasale, intrapéritonéale, sous-cutanée, par sonde, sublinguale, intraveineuse ou orale.

38. Composition pharmaceutique selon l'une des revendications 19, 22, 27 ou 32, dans laquelle la composition est formulée pour une administration orale.

39. Composition pharmaceutique, en particulier telle qu'elle est définie dans au moins l'une des revendications 19 à 38, destinée à être utilisée en tant que médicament dans un traitement visant à réduire la mort des cellules neuronales, comprenant la mise en contact des cellules neuronales avec un polypeptide du Facteur Neurotrophique Dépendant de l'Activité (ADNF) en quantité suffisante pour empêcher la mort des cellules neuronales, dans laquelle le polypeptide ADNF est un élément sélectionné parmi le groupe composé de :
(a) un polypeptide ADNF I comprenant un site central actif ayant la séquence d'acides aminés suivante : Ser-Ala-Leu-Leu-Arg-Ser-Ile-Pro-Ala (N° d'ID de SÉQ : 1) ;
(b) un polypeptide ADNF III ayant la séquence d'acides aminés suivante : Asn-Ala-Pro-Val-Ser-Ile-Pro-Gln (N° d'ID de SÉQ : 2) ; et
(c) un mélange du polypeptide ADNF I du point (a) et du polypeptide ADNF III du point (b) ;
dans laquelle au moins l'un des polypeptides ADNF I et ADNF III comprend un site central actif comprenant au moins un acide aminé D.

40. Composition pharmaceutique destinée à être utilisée en tant que médicament dans un traitement selon la revendication 39, dans laquelle les cellules neuronales sont sélectionnées parmi le groupe composé des neurones de la moelle épinière, des neurones de l'hippocampe, des neurones du cortex cérébral et des neurones cholinergiques.

41. Composition pharmaceutique destinée à être utilisée en tant que médicament dans un traitement selon la revendication 39, dans laquelle la mort des cellules neuronales se produit chez un patient infecté par un virus d'immunodéficience, en particulier dans lequel le virus d'immunodéficience est le virus de l'immunodéficience humaine.

42. Composition pharmaceutique destinée à être utilisée en tant que médicament dans un traitement selon la revendication 39, dans laquelle la mort des cellules neuronales est associée à une excito-toxicité induite par stimulation au N-méthyle D-aspartate.

43. Composition pharmaceutique destinée à être utilisée en tant que médicament dans un traitement selon la revendication 39, dans laquelle la mort des cellules neuronales est induite par le peptide bêta-amyloïde chez un patient atteint de la maladie d'Alzheimer.

44. Composition pharmaceutique destinée à être utilisée en tant que médicament dans un traitement selon la revendication 39, dans laquelle la mort des cellules neuronales est induite par un blocage cholinergique chez un patient atteint de la maladie d'Alzheimer, le blocage cholinergique entraînant des troubles de l'apprentissage.

45. Composition pharmaceutique, en particulier telle qu'elle est définie dans au moins l'une des revendications 19 à 38, destinée à être utilisée en tant que médicament dans un traitement du stress oxydatif chez un patient, comprenant l'administration au patient d'un polypeptide du Facteur Neurotrophique Dépendant de l'Activité (ADNF) en quantité suffisante pour réduire le stress oxydatif, dans laquelle le polypeptide ADNF est un élément sélectionné parmi le groupe composé de :
(a) un polypeptide ADNF I comprenant un site central actif ayant la séquence d'acides aminés suivante : Ser-Ala-Leu-Leu-Arg-Ser-Ile-Pro-Ala (N° d'ID de SÉQ : 1) ;
(b) un polypeptide ADNF III ayant la séquence d'acides aminés suivante : Asn-Ala-Pro-Val-Ser-Ile-Pro-Gln (N° d'ID de SÉQ : 2) ; [?] et
(c) un mélange du polypeptide ADNF I du point (a) et du polypeptide ADNF III du point (b) ;
dans laquelle au moins l'un des polypeptides ADNF I et ADNF III comprend un site central actif comprenant au moins un acide aminé D.

46. Composition pharmaceutique, en particulier telle qu'elle est définie dans au moins l'une des revendications 19 à 38, destinée à être utilisée en tant que médicament dans un traitement visant à réduire un état associé au syndrome d'alcoolisme foetal chez un sujet exposé à l'alcool *in utero,* comprenant l'administration au sujet d'un polypeptide du Facteur Neurotrophique Dépendant de l'Activité (ADNF) en quantité suffisante pour réduire l'état associé au syndrome d'alcoolisme foetal, dans laquelle le polypeptide ADNF est un élément sélectionné parmi le groupe composé de :
(a) un polypeptide ADNF I comprenant un site central actif ayant la séquence d'acides aminés suivante : Ser-Ala-Leu-Leu-Arg-Ser-Ile-Pro-Ala (N° d'ID de SÉQ : 1) ;
(b) un polypeptide ADNF III ayant la séquence d'acides aminés suivante : Asn-Ala-Pro-Val-Ser-Ile-Pro-Gln (N° d'ID de SÉQ : 2) et
(c) un mélange du polypeptide ADNF I du point (a) et du polypeptide ADNF III du point (b) ;
dans laquelle au moins l'un des polypeptides ADNF I et ADNF III comprend un site central actif comprenant au moins un acide aminé D.

47. Composition pharmaceutique destinée à être utilisée en tant que médicament dans un traitement selon la revendication 46, dans laquelle l'état est sélectionné parmi le groupe composé de : une diminution du poids corporel d'un sujet, une diminution du poids cérébral d'un sujet, une diminution du niveau d'ARNm VIP d'un sujet ; et mort d'un sujet *in utero.*

48. Utilisation de la composition pharmaceutique, en particulier telle qu'elle est définie dans au moins l'une des revendications 19 à 38, pour la fabrication d'un médicament destiné à réduire la mort des cellules neuronales selon au moins l'une des revendications 39 à 44.

49. Utilisation de la composition pharmaceutique, en particulier telle qu'elle est définie dans au moins l'une des revendications 19 à 38, pour la fabrication d'un médicament destiné au traitement du stress oxydatif chez un patient selon la revendication 45.

50. Utilisation de la composition pharmaceutique, en particulier telle qu'elle est définie dans au moins l'une des revendications 19 à 38, pour la fabrication d'un médicament pour un traitement destiné à réduire un état associé au syndrome d'alcoolisme foetal chez un sujet exposé à l'alcool *in utero* selon au moins l'une des revendications 46 à 47.
